(19) Europäisches Patentamt
European Patent Office
Office européen des brevets

(11) EP 4 097 166 B1

(12) **EUROPEAN PATENT SPECIFICATION**

(45) Date of publication and mention of the grant of the patent:
**03.04.2024 Bulletin 2024/14**

(21) Application number: **21703860.3**

(22) Date of filing: **01.02.2021**

(51) International Patent Classification (IPC):
***C08G 63/08*** *(2006.01)* ***C08G 65/34*** *(2006.01)*
***C08G 65/48*** *(2006.01)* ***C08G 83/00*** *(2006.01)*
***C08L 71/08*** *(2006.01)* ***C08G 63/685*** *(2006.01)*
***C08G 63/688*** *(2006.01)* ***C08G 81/00*** *(2006.01)*

(52) Cooperative Patent Classification (CPC):
(C-Sets available)
**C08G 65/34; C08G 63/08; C08G 63/6852; C08G 63/6882; C08G 65/48; C08G 81/00; C08G 83/006; C08L 71/08;** C08G 2650/54 (Cont.)

(86) International application number:
**PCT/EP2021/052303**

(87) International publication number:
**WO 2021/152171 (05.08.2021 Gazette 2021/31)**

(54) **MICELLAR COMPOSITION FROM AN AMPHIPHILIC COPOLYMER FOR TUMOR THERAPY**

MIZELLARE ZUSAMMENSETZUNG AUS EINEM AMPHIPHILEN COPOLYMER ZUR TUMORTHERAPIE

COMPOSITION MICELLAIRE À PARTIR D’UN COPOLYMÈRE AMPHIPHILE POUR LE TRAITEMENT DE TUMEURS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **31.01.2020 EP 20154944**

(43) Date of publication of application:
**07.12.2022 Bulletin 2022/49**

(73) Proprietor: **Freie Universität Berlin**
**14195 Berlin (DE)**

(72) Inventors:
- **HAAG, Rainer**
  **12209 Berlin (DE)**
- **SCHIRNER, Michael**
  **13158 Berlin (DE)**
- **BRAATZ, Daniel**
  **13583 Berlin (DE)**
- **ZHONG, Yinan**
  **211198 Nanjing (CN)**
- **DIMDE, Mathias**
  **13351 Berlin (DE)**

(74) Representative: **Maikowski & Ninnemann**
**Patentanwälte Partnerschaft mbB**
**Postfach 15 09 20**
**10671 Berlin (DE)**

(56) References cited:
- **ZHONG YINAN ET AL: "Micelles with Sheddable Dendritic Polyglycerol Sulfate Shells Show Extraordinary Tumor Targetability and Chemotherapy in Vivo.", ACS APPLIED MATERIALS & INTERFACES, vol. 8, no. 41, 19 October 2016 (2016-10-19), pages 27530-27538, XP002799745, ISSN: 1944-8252**
- **DU FANG ET AL: "Development of biodegradable hyperbranched core-multishell nanocarriers for efficient topical drug delivery", JOURNAL OF CONTROLLED RELEASE, vol. 242, 1 July 2016 (2016-07-01), pages 42-49, XP029809677, ELSEVIER ISSN: 0168-3659, DOI: 10.1016/J.JCONREL.2016.06.048**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 097 166 B1

- **GAO X ET AL: "Synthesis and physicochemical characterization of a novel amphiphilic polylactic acid-hyperbranched polyglycerol conjugate for protein delivery", JOURNAL OF CONTROLLED RELEASE, vol. 140, no. 2, 3 December 2009 (2009-12-03), pages 141-147, XP026741910, ELSEVIER ISSN: 0168-3659, DOI: 10.1016/J.JCONREL.2009.08.003 [retrieved on 2009-08-13]**

(52) Cooperative Patent Classification (CPC): (Cont.)

C-Sets
**C08L 71/08, C08L 67/04**

## Description

The present invention relates to an amphiphilic copolymer according to the preamble of claim 1, to a micelle comprising such an amphiphilic copolymer according to the preamble of claim 9, to uses of such a micelle according to claims 10 to 12 as well as to a method for manufacturing an amphiphilic copolymer according to the preamble of claim 13.

The application of drugs to a patient by means of targeted delivery of the active ingredient has been a key area of research for decades and has been fueled by the many recent developments in polymer science. Especially in the field of new anti-tumor drugs, research has been focused on targeted delivery of therapeutic molecules to the localized tumor or tumor metastasis. Moreover, at the time point of clinical diagnosis of a tumor disease, the size or volume of the primary tumor is often in the range of 1 to 5 ml/cm$^3$. In many cases, the small tumor size only accounts for 1/50,000 of the total body volume. Considering the small size of the primary tumor at diagnosis, therapeutic drugs must be applied in a significant access to reach sufficient concentration in the tumor tissue. Furthermore, due to the impaired blood supply of many tumors, effective drug concentrations are usually lower than in healthy tissue. In this regard, it is of utmost importance to search for new means of targeted delivery of drugs to increase drug efficacy and safety.

Several strategies were followed during the last three decades to spare healthy tissues and organs from non-intended drug effects. Predominantly, drug research was focused on new drug targets that promised a disease-specific expression of the target mechanism. With respect to the discovery of signal transduction mechanisms in proliferating and activated cells, numerous new targets have been identified. Unfortunately, many tumors exhibit effective resistance mechanisms after treatment with highly specific anti-tumor drugs. Furthermore, with few exceptions therapeutic attack of most newly discovered drug targets remained less effective, since the therapeutic molecule must possess the physicochemical properties to penetrate tissues, membrane barriers and cells to reach the target, when the target is expressed inside of cells. Thus, much effort has been devoted improving the bioavailability of clinically established therapeutic drugs by chemical modification or pharmaceutical formulations of the therapeutic molecules.

A proven approach according to prior art is the chemical conjugation of therapeutic drugs to molecules having targeting properties. Very often, peptides or proteins with high binding affinity to certain receptors on the tumor cell membrane are used to direct the therapeutic drug to the tumor target. Despite relevant therapeutic effects in a few model systems, the broader application of chemical drug conjugates is hampered for several reasons. On the one hand, most known tumor targets are variably expressed and downregulated under therapy. In this respect, chemical drug conjugates lose their binding affinity during therapy. On the other hand, the release of the drug from the carrying molecules is often impaired und incomplete which leads to ineffective drug concentration.

With respect to poorly soluble therapeutic and diagnostic drugs, much attention was given pharmaceutical formulations using biocompatible amphiphilic polymers. In general, the advantage of pharmaceutical formulations is the controlled release of the drug as active pharmaceutical ingredients (API). Respective amphiphilic polymers for drug encapsulation are polymers having both hydrophilic and hydrophobic properties. A proven approach to synthesize a copolymer having both hydrophilic and hydrophobic properties is the chemical conjugation of a hydrophilic polymer such as polyethylene glycol (PEG) and a hydrophobic polymer such as polylactic acid (PLA) or polycaprolactone PCL. Resulting amphiphilic copolymers may form micelles which are aggregates of molecules in a colloidal system. Typical micelles in aqueous solution have a size of 1 nm to 1000 nm. The self-assembling organization of micelles in aqueous solution results from interaction of the hydrophilic head regions of the copolymer in contact with surrounding solvent, and sequestering the hydrophobic single-tail, core regions in the micelle center. Hereby, the hydrophobic core region serves as drug storage space for poorly water soluble, hydrophobic drugs.

Recently, copolymer systems have been successfully proven as nanosized carriers of compound for diagnosis and therapy of diseases. Especially, PEG-based micelle forming, amphiphilic copolymers have been demonstrated to be suited for pharmaceutical formulation of poorly soluble drugs. The drugs are typically encapsulated in a copolymer micelle. The pharmaceutical usability of micelle-forming copolymers is determined by their capacity to dissolve poorly water-soluble drugs and their biodegradability and safety following application into human. However, the therapeutic efficacy of drug loaded micelles is determined by a fine-tuned balance of micelles stability during blood circulation and micelle degradation und respective drug release in the tumor tissue environment. Both stability and degradability at the target tissue are indispensable for overall therapeutic efficacy. For example, a micelle that is easily degradable at the target tissue to release the encapsulated drug is of minor clinical usability if the micellar drug formulation is not stable during circulation. Those micelles will eventually release highly toxic drug before reaching the disease target. Once highly toxic drugs are released before reaching the disease target, they may lead to unintentional toxic effects on healthy tissue. Moreover, instable micelles are not suited to reach high drug concentrations at the tumor target. In this regard, there is a need for micellar copolymer drug formulations having necessary stability during blood circulation.

Targeting of drug-loaded copolymer micelles (e.g., targeted to a tissue or cell type or targeted to a specific diseased tissue but not to normal tissue) is desirable because it reduces the amount of a drug present in tissues of the body that are not targeted. This is of relevance when treating a condition such as cancer or life-threatening inflammation where it is desirable that a potentially cytotoxic dose of the drug is delivered to the diseased cells without killing the

surrounding healthy tissue. Targeted drug delivery is proven to reduce the undesirable and sometimes life-threatening side effects common in anticancer therapy. In addition, targeting may allow drugs to reach certain tissues they would otherwise be unable to reach without a targeted polymer micelle.

Novel pharmaceutical approaches have been established in order to deliver poorly soluble drugs into patients. Polymer systems based on amphiphilic copolymers have been proven to encapsulate drugs and increase bioavailability after parental application. Most recently, intrinsically targeted polymer systems based on the tumor and inflammation targeted dendritic polyglycerol sulfate (dPGS) has been published (Zhong et al., 2016; Reference 6 in the list of References). The tumor and inflammation targeting property of dPGS is well established in-vitro and in-vivo. To achieve proper accumulation of dPGS-based polymer system in the tumor area, sustained circulation in the blood up to 6 hours following parental application is required.

Albeit the targeted drug delivery by dPGS-based copolymer micelles have been demonstrated by Zhong et al., translation into clinical application is hampered by several disadvantages of this prior art technical solution.

First, drug-loaded micelles formed by the reductive cleavable dPGS-SS-PCL copolymer of Zhong et al. showed early drug release to a relevant extent. To be more precise, encapsulated drugs are released from the polymer micelle as indicated by in-vitro incubation in phosphate-buffered saline (PBS buffer) as well as shortly after parental application in tumor-bearing animals. In-vitro incubation in PBS buffer leads to a 28 % spontaneous leaching of the drug within 24 hours which indicates a certain degree of instability of the pharmaceutical form. In tumor-bearing mice, early leaching of the encapsulated drug can be studied using fluorescent dyes. An instable micelle formulation leads to fluorescence signal increase in all parts of the organism early after injection of the micellar dye formulation. In general, instable copolymer micelles lead to a relevant decrease of the drug concentration in the tumor and increase of drug distribution within healthy tissue.

Second, pharmaceutical compositions based on prior art techniques such as disclosed in Zhong et al. are hampered by a synthetic route which is contaminated with hazardous byproducts and not suited for scale-up to produce enough drug substance for reasonable costs (1 mg micelle composition produced according to Zhong et al. costs at least 100 Euros).

Third, prior art dPGS-based copolymer micelles are present in form of aqueous solutions which are not suited for long-term storage. So far, no pharmaceutical formulation is known to enable medical use for long time and under convenient storage conditions.

Accordingly, a need exists to develop micellar polymer systems demonstrating improved efficacy and stability without drug leaching before reaching the target. At the same time, such micellar polymer systems should be manufactured at reasonable costs.

This need is addressed with an amphiphilic copolymer having the features of claim 1. Such an amphiphilic copolymer comprises a first block, a second block and a linker covalently linking the first block with the second block.

The first block is a hydrophilic dendritic polyglycerol derivative having a polyglycerol backbone and carrying a plurality of sulfate or sulfonate residues substituting hydroxyl groups of the polyglycerol backbone. In an embodiment, the polyglycerol derivative carries a plurality of sulfate residues as substituents of hydroxyl groups of the polyglycerol backbone, i.e., the polyglycerol derivative is a polyglycerol sulfate.

The second block is a hydrophobic block comprising a polymer chosen from the group consisting of polycaprolactone, a polylactic acid polymer, and a copolymer of lactic acid and glycolic acid. In an embodiment, the hydrophobic block consists of this polymer.

According to an aspect of the invention, the linker comprises a hydrocarbon having at least six consecutive methylene ($CH_2$) residues and a cleavable entity. Furthermore, the linker is devoid of a triazole-containing residue resulting from a reaction between an alkyne and an azide.

To give an example, the linker of the amphiphilic copolymer disclosed by Zhong et al. is made by a cycloaddition between an azide and a cyclooctyne residue (so-called click chemistry). The resulting structural feature of the linker comprises a triazole resulting from this reaction (cf. scheme 2 of Zhong et al).

It was surprisingly found that a linker being devoid of such structural motive is much more stable than the linker disclosed by Zhong et al. This results in a higher stability of a micelle built up by the novel amphiphilic copolymers. Such higher stability results in lower leaching of drugs encapsulated in a micelle built up from the novel amphiphilic copolymer.

To be more precise, the new linker structure enables particularly stable encapsulation of hydrophobic drugs and cost-effective synthesis. While micelles made from a dPGS-copolymer according to prior art show a spontaneous leaching of the encapsulated drugs to a degree of 28 % within 24 hours, the amphiphilic copolymers described herein show no or very low spontaneous drug leaching for 24 hours incubation in buffer media. Furthermore, the amphiphilic copolymer described herein demonstrates a significantly improved dilution stability as indicated by the critical micelle concentration (CMC) value. The stability of a micelle after dilution in aqueous media is indicated by the CMC and is a prediction for stability of micellar drug formulations in the blood circulation. The lower the CMC, the higher the stability in the blood circulation. While micelle-forming dPGS-copolymers according to prior art demonstrate a CMC in the range of 5 to 6 μg/ml, micelles built up from the amphiphilic copolymer described herein exhibit a factor 10 lower CMC indicating

higher blood circulatory stability. Micellar drug formulation characterized by a very low drug leakage have a lower risk of toxicity related to the action of the free drug.

The linker may form intermolecular interaction with other linker molecules, e.g., to stabilize a drug-loaded micelle built up from the amphiphilic copolymer and to prevent drug release from the hydrophobic core of such a micelle.

Due to the cleavable entity in the linker, it is nonetheless possible to disintegrate the stability of the linker by changing the redox potential of the surrounding environment of the amphiphilic copolymer.

In an embodiment, the cleavable entity of the linker is a redox-sensitive entity. Such a redox-sensitive entity can be cleaved by changing the redox properties. E.g., if a redox-sensitive entity is transferred from a first environment to a second environment and if the redox potential of the second environment is sufficiently higher or lower than that of the first environment, the redox-sensitive entity is cleaved.

In an embodiment, the redox-sensitive entity is a disulfide bridge. Such disulfide bridge is opened in a reducing environment so that the linker is cleaved in such environment. This results in a collapse of a micelle built up from the amphiphilic copolymer and consequently to a release of a drug or other agent encapsulated in such micellar.

In an embodiment, the cleavable entity of the linker is a pH-cleavable entity. Such pH-cleavable entity can be cleaved upon a pH change of the environment. E.g., if a pH-cleavable entity is transferred from a first environment to a second environment and if the pH value of the second environment is sufficiently higher or lower than that of the first environment, the pH-cleavable entity is cleaved.

In an embodiment, the pH-cleavable cleavable entity is an imine, an oxime, a hydrazone, or an acetal.

A pH-cleavable entity in form of an imine or an acetal can be particularly simply introduced into the linker, e.g., with a protected aldehyde via a thiol-ene synthesis. After deprotecting the aldehyde, an imine or acetal results.

In an embodiment, the amphiphilic copolymer corresponds to the following general formula:

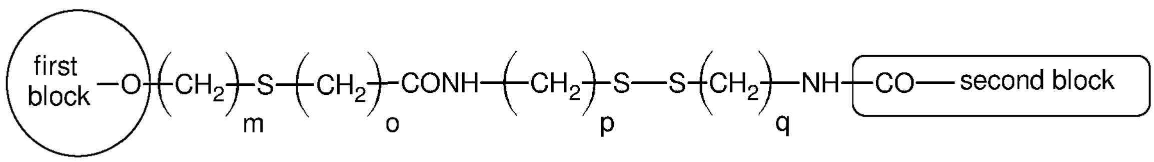

wherein

- m = 6 to 20, in particular 7 to 19, in particular 8 to 18, in particular 9 to 17, in particular 10 to 16, in particular 11 to 15, in particular 12 to 14, in particular 11 to 13,
- o = 0 to 4, in particular 2 or 3,
- p = 0 to 4, in particular 2 or 3,
- q = 0 to 4, in particular 2 or 3.

It was surprisingly found that an amphiphilic copolymer having a molecular weight of greater 5,000 g/mol for the hydrophilic dendritic polyglycerol derivative (e.g., a dPGS polymer) (first block) and a molecular weight of greater 5,000 g/mol for the hydrophobic polymer such as PCL, PLA and PLGA (second block) are especially suited for encapsulation of hydrophobic drugs and show superior pharmaceutical properties compared to the dPGS-copolymer known from prior art.

In an embodiment, the molecular weight of the first block lies in a range of 5000 to 100,000 g/mol, in particular 6000 to 90,000 g/mol, in particular 7000 to 80,000 g/mol, in particular 8000 to 70,000 g/mol, in particular 9000 to 60,000 g/mol, in particular 10,000 to 50,000 g/mol, in particular 15,000 to 40,000 g/mol, in particular 20,000 to 30,000 g/mol. A range of 5000 to 15,000 g/mol is particularly appropriate.

In an embodiment, the molecular weight of the second block lies in a range of 5000 to 100,000 g/mol, in particular 6000 to 90,000 g/mol, in particular 7000 to 80,000 g/mol, in particular 8000 to 70,000 g/mol, in particular 9000 to 60,000 g/mol, in particular 10,000 to 50,000 g/mol, in particular 15,000 to 40,000 g/mol, in particular 20,000 to 30,000 g/mol. A range of 5000 to 15,000 g/mol is particularly appropriate.

In an embodiment, the degree of substitution (sulfation or sulfonation) of the polyglycerol backbone is between 10 % and 100 %, in particular between 15 % and 95 %, in particular between 20 % and 90 %, in particular between 25 % and 85 %, in particular between 30 % and 80 %, in particular between 35 % and 75 %, in particular between 40 % and 70 %, in particular between 45 % and 65 %, in particular between 50 % and 60 %, in particular between 55 % and 58 %, (in each case including the upper and lower limits). A very well-suited degree of substitution is between 50 % and 100 %. Another very well-suited degree of substitution is between 70 % and 100 %. Another very well-suited degree of substitution is between 85 % and 100 %. Another very well-suited degree of substitution is between 90 % and 100 % (in each case including the upper and lower limit).

Depending on the choice of the polymerization conditions the polyglycerol backbone reaches a branching degree and an arbitrarily adjustable molecular weight with narrow polydispersities. According to the present invention, polyglycerol backbones with a branching degree of more than 0 up to 100 % may be used. In an embodiment, highly branched structures are used, in particular with a branching degree of 20 to 90 %, in particular of 30 to 80 %, in particular of 40 to 70 %, in particular of 50 to 60 %, in particular having a branching degree of around 60 % (55 % to 65 %).

In an embodiment, the second block (hydrophobic core) comprises or consists of polycaprolactone, collectively referred to herein as "PCL."

In an embodiment, the second block comprises or consists of a polylactic acid polymer.

In an embodiment, the polylactic acid polymer is chosen from the group consisting of poly-L-lactic acid (poly-L-lactide), poly-D-lactic acid (poly-D-lactide), and poly-D,L-lactic acid (poly-D,L-lactide), collectively referred to herein as "PLA."

In an embodiment, the second blocker comprises or consists of a copolymer of lactic acid and glycolic acid (PLGA). PLGA is a biocompatible and biodegradable copolymer, and various forms of PLGA are characterized by the ratio of lactic acid:glycolic acid. Lactic acid can be L-lactic acid, D-lactic acid, or D,L-lactic acid. The degradation rate of PLGA can be adjusted by altering the lactic acid to glycolic acid ratio. In an embodiment, the PLGA is characterized by a lactic acid:glycolic acid ratio of approximately 85:15, approximately 75:25, approximately 60:40, approximately 50:50, approximately 40:60, approximately 25:75, or approximately 15:85. Suited lactic acid:glycolic acid ratio ranges are ranges from 85:15 to 15:85 or any other ranges that can be built up from the before-mentioned ratios.

In an embodiment, the linker comprises 6 to 20 consecutive methylene residues (e.g., a $C_6$-$C_{20}$ alkyl), in particular 7 to 19, in particular 8 to 18, in particular 9 to 17, in particular 10 to 16, in particular 11 to 15, in particular 12 to 14, in particular 11 to 13 consecutive methylene residues.

In an embodiment the polymer of the second block comprises 10 to 200 repeating units, in particular 15 to 190 repeating units, in particular 20 to 180 repeating units, in particular 25 to 170 repeating units, in particular 30 to 160 repeating units, in particular 40 to 150 repeating units, in particular 50 to 140 repeating units, in particular 60 to 130 repeating units, in particular 70 to 120 repeating units, in particular 80 to 110 repeating units, in particular 90 to 100 repeating units.

In an embodiment, the second block comprises or consists of PCL and has 20 to 80 repeating units, in particular 30 to 75 repeating units, in particular 35 to 70 repeating units, in particular 40 to 65 repeating units, in particular 50 to 55 repeating units. To give a specific example, a hydrophobic block consisting of PCL and having 35 repeating units has a molecular weight of approximately 4000 g/mol.

In an embodiment, the second block comprises or consists of PLGA and has 10 to 50 lactide repeating units and 10 to 40 glycolide repeating units, in particular 15 to 45 lactide repeating units and 15 to 35 glycolide repeating units, in particular 20 to 40 lactide repeating units and 20 to 30 glycolide repeating units, in particular 25 to 35 lactide repeating units and 25 to 30 glycolide repeating units. To give a specific example, a hydrophobic block consisting of PLGA and having 17 lactide repeating units and 14 glycolide repeating units has a molecular weight of approximately 4000 g/mol.

In an embodiment, the second block comprises or consists of PLA and has 20 to 70 repeating units, in particular 25 to 65 repeating units, in particular 30 to 60 repeating units, in particular 35 to 55 repeating units, in particular 40 to 50 repeating units. To give a specific example, a hydrophobic block consisting of PLA and having 28 repeating units has a molecular weight of approximately 4000 g/mole.

In an aspect, the present invention relates to a micelle comprising a plurality of molecules of at least one amphiphilic copolymer according to the preceding explanations. Such a micelle is autonomously formed upon transferring the amphiphilic copolymer into an aqueous solution or by reducing the content of organic solvent in a solvent mixture comprising an organic solvent and an aqueous solvent. Since the first block (comprising a polyglycerol derivative) is hydrophilic, it will be oriented in an aqueous solution towards an outside of the micelle. In contrast, the hydrophobic second block will be oriented towards an inside of the micelle in an aqueous environment. Thus, the amphiphilic copolymer behaves like a detergent in an aqueous solution and facilitates encapsulating hydrophobic agents in its interior (next to the second block).

In an embodiment, the micelle comprises only amphiphilic copolymers of a single type. In another embodiment, the micelle comprises amphiphilic copolymers of at least 2 different types, in particular of 2 to 10, in particular 3 to 9, in particular 4 to 8, in particular 5 to 7 different types.

In an aspect, the present invention relates to the use of a micelle as explained in the preceding paragraph encapsulating an agent in an interior of the micelle. The agent is typically a hydrophobic agent. The agent can, e.g., be a drug, i.e., a pharmaceutically active compound.

In an aspect, the present invention relates to the medical use of the micellar composition comprising a micelle according to the preceding explanations and an agent encapsulated in an interior of the micelle, i.e. to the use of the micellar composition as medicament. Thereby, the encapsulated agent is an agent of medical relevance, e.g., a diagnostic or therapeutic agent like a drug, i.e. a pharmaceutically active compound.

In an aspect, the invention relates to the use of a micellar composition, comprising a micelle according to the

preceding explanations and an anti-tumor agent encapsulated in an interior of the micelle, in treating a tumor.

Thus, the micellar composition is, in this embodiment, particularly appropriate in the treatment or prevention or amelioration of one or more symptoms of cancer, particularly cancers that express proteins with high binding affinity to dPGS, including, but not limited to, breast cancer, colon cancer, glioma, renal cancer, hepatocellular cancer, lung cancer, head- and neck prostate cancer, non-small cell lung cancer, colorectal cancer, pancreatic cancer, melanoma and leukemia.

In an embodiment, a micelle (comprising an amphiphilic copolymer according to the preceding explanations) encapsulates a therapeutic moiety, i.e., a moiety that has a therapeutic or prophylactic effect when given to a subject. Examples of therapeutic moieties to be used with the polymer micelles of the present invention include antineoplastic or cytostatic agents or other agents with anticancer properties, or a combination thereof. The term "subject" encompasses humans and animals (in particular non-human mammals). Thus, the instant invention relates in an aspect to a method of treating a human or an animal (in particular a non-human mammal).

In an embodiment, the micelle acts as a nanocarrier, i.e., the micelle has a characteristic dimension of less than about 1 micrometer, wherein the characteristic dimension of a micelle is the diameter of a perfect sphere having the same volume as the micelle. For example, the micelle may have a characteristic dimension of the less than about 300 nm, less than about 200 nm, less than about 150 nm, less than about 100 nm, less than about 50 nm, less than about 30 nm, less than about 10 nm, less than about 3 nm, or less than about 1 nm in some cases. In an embodiment, the micelle has a diameter of 1 nm to 300 nm or of any other range that can be built up from the precedingly mentioned values, such as a range of 30 nm to 100 nm.

In an embodiment, the micelle contains a therapeutic agent. Examples of therapeutic agents include, but are not limited to, a chemotherapeutic agent, a cell signaling inhibitor, a radioactive agent, a nucleic acid-based agent, a lipid-based agent, a carbohydrate-based agent, a natural small molecule, or a synthetic small molecule.

Very surprisingly, the micellar composition comprising a micelle according to the preceding explanations and a drug (in particular a drug having anti-proliferative activity) encapsulated in an interior of the micelle demonstrated a significant improvement of anti-proliferative activity compared to prior art dPGS-copolymer micelles. While Zhong et al. found a factor 7 lower anti-proliferative activity in tumor cell lines of the micellar dPGS-copolymer formulation, the presently described micellar composition is at least equally or even more anti-proliferatively active than the free drug. Therefore, the micellar composition is surprisingly characterized by the combination of very low, spontaneous drug leakage and high anti-proliferative action on tumor cells.

In a particular embodiment, the molecular weight of the polymers of the drug encapsulating micelle are optimized for effective treatment of cancer. For example, the molecular weight of the polymer influences micelle stability (particularly when the molecular weight of a biodegradable polymer is adjusted), solubility, water uptake, and drug release behavior (e.g. "reductive cleavage"). As a further example, the molecular weight of the polymer can be adjusted such that the blood circulation of micelles in the subject being treated is within a reasonable period of time (ranging from a few minutes to 1-2 hours, 3-4 hours, 5-6 hours, 7-8 hours, etc.). In an embodiment, the dendritic polyglycerol derivative of the first block has a molecular weight of 5,000-100,000 g/mol, e.g., 8,000-20,000 g/mol, e.g., 10,000-20,000 g/mol, and the hydrophobic polymer of the second block has a molecular weight of 5,000-100,000 g/mol, e.g., 6,000-20,000 g/mol, e.g., 7,000-10,000 g/mol. Other suited molecular weight ranges for the first and/or the second block are those already indicated above, i.e., 5000 to 100,000 g/mol, in particular 6000 to 90,000 g/mol, in particular 7000 to 80,000 g/mol, in particular 8000 to 70,000 g/mol, in particular 9000 to 60,000 g/mol, in particular 10,000 to 50,000 g/mol, in particular 15,000 to 40,000 g/mol, in particular 20,000 to 30,000 g/mol.

### Therapeutic Agents

According to embodiments, any agents ("payload"), including, for example, therapeutic agents (e.g. anti-cancer agents), diagnostic agents (e.g. contrast agents; radionuclides; and fluorescent, luminescent, and magnetic moieties), prophylactic agents (e.g. vaccines), and/or nutraceutical agents (e.g. vitamins, minerals, etc.) may be delivered by a micelle formed by the amphiphilic copolymer as described herein. Exemplary agents to be include, but are not limited to, small molecules (e.g. cytotoxic agents), nucleic acids (e.g., siRNA, RNAi, and mircoRNA agents), proteins (e.g. antibodies), peptides, lipids, carbohydrates, hormones, metals, radioactive elements and compounds, drugs, vaccines, immunological agents, etc., and/or combinations thereof. In some embodiments, the agent to be delivered is an agent useful in the treatment of cancer. For instance, the targeting property of dPGS based copolymers may target or cause the micelle to become localized at specific portions within a subject, and the payload may be delivered to those portions. In a particular embodiment, the drug or other payload may is released from the polymer micelles following local interaction of the dPGS with particular binding molecules (e.g., proteins and peptides such as bFGF, TGF, IL-1, L-selectin). The term "controlled release" (and variants of that term) as used herein (e.g., in the context of "controlled-release system") is generally meant to encompass release of a substance (e.g., a drug) at a selected site or otherwise controllable in rate, interval, and/or amount by certain condition at the targeting site such as pH value, oxygen saturation etc. Controlled

release encompasses, but is not necessarily limited to, substantially continuous release, patterned release (e.g., intermittent release over a period of time that is interrupted by regular or irregular time intervals), and release of a bolus of a selected substance (e.g., as a predetermined, discrete amount if a substance over a relatively short period of time (e.g., a few seconds or minutes)).

All explanations given above and below for a dendritic polyglycerol sulfate (dPGS) are likewise applicable and transferable to a dendritic polyglycerol sulfonate (sometimes also abbreviated as dPGS). Both dendritic polyglycerol sulfate and dendritic polyglycerol sulfonate are a dendritic polyglycerol derivative within the definitions of the present disclosure.

For example, the targeting property of dPGS based micelles may cause the nanocarrier to become localized to a tumor, a disease site, a tissue, an organ, a type of cell, etc. within the body of a subject, depending on the expression of one or more dPGS binding molecules. For example, the protein L-selectin is a well-characterized binding molecule of dPGS and may become expressed in many cancers. While in some cancers, expression of the dPGS binding molecules is limited to neovasculature, some others cancer show expression of dPGS binding molecules in both neovasculature and tumor cells.

In an embodiment, the payload is a drug or a combination of more than one drug. Exemplary therapeutic agents include chemotherapeutic agents such as doxorubicin (adriamycin), gemcitabine (gemzar), daunorubicin, procarbazine, mitomycin, cytarabine, etoposide, methotrexate, 5-fluorouracil (5-FU), vinblastine, vincristine, vindesine, bleomycin, paclitaxel (taxol), docetaxel (taxotere), aldesleukin, asparaginase, busulfan, carboplatin, cladribine, camptothecin, CPT-11, 10-hydroxy-7-ethylcamptothecin (SN38), dacarbazine, S-I capecitabine, ftorafur, 5' deoxyfluorouridine, UFT, eniluracil, deoxycytidine, 5-azacytosine, 5-azadeoxycytosine, allopurinol, 2-chloroadenosine, trimetrexate, aminopterin, methylene-10-deazaminopterin (MDAM), oxaplatin, picoplatin, tetraplatin, satraplatin, platinum-DACH, ormaplatin, CI-973, JM-216, and analogs thereof, bortezomib, epirubicin, etoposide phosphate, 9-aminocamptothecin, 10,11-methylenedioxycamptothecin, karenitecin, 9-nitrocamptothecin, TAS 103, L-phenylalanine mustard, ifosphamidemefosphamide, perfosfamide, trophosphamide carmustine, semustine, epothilones A-E, tomudex, 6-mercaptopurine, 6-thioguanine, amsacrine, etoposide phosphate, karenitecin, acyclovir, valacyclovir, ganciclovir, amantadine, rimantadine, lamivudine, zidovudine, bevacizumab, trastuzumab, rituximab, 5-Fluorouracil, and combinations thereof.

Non-limiting examples of potentially suitable drugs include anti-cancer agents, including, for example, docetaxel, mitoxantrone, and mitoxantrone hydrochloride. In another embodiment, the payload may be an anti-cancer drug such as 20-epi-1, 25 dihydroxyvitamin D3,4-ipomeanol, 5-ethynyluracil, 9-dihydrotaxol, abiraterone, acivicin, aclarubicin, acodazole hydrochloride, acronine, acylfiilvene, adecypenol, adozelesin, aldesleukin, all-tk antagonists, altretamine, ambamustine, ambomycin, ametantrone acetate, amidox, amifostine, aminoglutethimide, aminolevulinic acid, amrubicin, amsacrine, anagrelide, anastrozole, andrographolide, angiogenesis inhibitors, antagonist D, antagonist G, antarelix, anthramycin, anti-dorsalizdng morphogenetic protein-1, antiestrogen, antineoplaston, antisense oligonucleotides, aphidicolin glycinate, apoptosis gene modulators, apoptosis regulators, apurinic acid, ARA-CDP-DL-PTBA, arginine deaminase, asparaginase, asperlin, asulacrine, atamestane, atrimustine, axinastatin 1, axinastatin 2, axinastatin 3, azacitidine, azasetron, azatoxin, azatyrosine, azetepa, azotomycin, baccatin III derivatives, balanol, batimastat, benzochlorins, benzodepa, benzoylstaurosporine, beta lactam derivatives, beta-alethine, betaclamycin B, betulinic acid, bFGF inhibitor, bicalutamide, bisantrene, bisantrene hydrochloride, bisazuidinylspermine, bisnafide, bisnafide dimesylate, bistratene A, bizelesin, bleomycin, bleomycin sulfate, BRC/ABL antagonists, breflate, brequinar sodium, bropirimine, budotitane, busulfan, buthionine sulfoximine, cactinomycin, calcipotriol, calphostin C, calusterone, camptothecin derivatives, canarypox IL-2, capecitabine, caraceraide, carbetimer, carboplatin, carboxamide-amino-triazole, carboxyamidotriazole, carest M3, carmustine, earn 700, cartilage derived inhibitor, carubicin hydrochloride, carzelesin, casein kinase inhibitors, castanosperrnine, cecropin B, cedefingol, cetrorelix, chlorambucil, chlorins, chloroquinoxaline sulfonamide, cicaprost, cirolemycin, cisplatin, cis-porphyrin, cladribine, clomifene analogs, clotrimazole, collismycin A, collismycin B, combretastatin A4, combretastatin analog, conagenin, crambescidin 816, crisnatol, crisnatol mesylate, cryptophycin 8, cryptophycin A derivatives, curacin A, cyclopentanthraquinones, cyclophosphamide, cycloplatam, cypemycin, cytarabine, cytarabine ocfosfate, cytolytic factor, cytostatin, dacarbazine, dacliximab, dactinomycin, daunorubicin hydrochloride, decitabine, dehydrodidemnin B, deslorelin, dexifosfamide, dexormaplatin, dexrazoxane, dexverapamil, dezaguanine, dezaguanine mesylate, diaziquone, didemnin B, didox, diethyhiorspermine, dihydro-5-azacytidine, dioxamycin, diphenyl spiromustine, docetaxel, docosanol, dolasetron, doxifluridine, doxorubicin, doxorubicin hydrochloride, droloxifene, droloxifene citrate, dromostanolone propionate, dronabinol, duazomycin, duocannycin SA, ebselen, ecomustine, edatrexate, edelfosine, edrecolomab, eflomithine, eflomithine hydrochloride, elemene, elsarnitrucin, emitefur, enloplatin, enpromate, epipropidine, epirubicin, epirubicin hydrochloride, epristeride, erbulozole, erythrocyte gene therapy vector system, esorubicin hydrochloride, estramustine, estramustine analog, estramustine phosphate sodium, estrogen agonists, estrogen antagonists, etanidazole, etoposide, etoposide phosphate, etoprine, exemestane, fadrozole, fadrozole hydrochloride, fazarabine, fenretinide, filgrastim, finasteride, flavopiridol, flezelastine, floxuridine, fluasterone, fludarabine, fludarabine phosphate, fluorodaunorunicin hydrochloride, fluorouracil, fluorocitabine, forfenimex, formestane, fosquidone, fostriecin, fostriecin sodium, fotemustine, gadolinium texaphyrin, gallium nitrate, galocitabine, ganirelix, gelatinase inhibitors, gemcitabine, gemcitabine hydrochloride, glutathione inhibitors, hepsulfam, heregulin, hexamethylene bisacetamide, hydroxyurea, hypericin, ibandronic acid, idarubicin, idarubicin hydrochloride, idoxifene, idramantone, ifosfamide, ihnofosine, ilomastat, imidazoacridones, imiquimod, immunostimulant peptides, insulin-like growth factor-1 receptor inhibitor, interferon agonists, interferon alpha-2A, interferon alpha-2B, interferon alpha-N1, interferon alpha-N3, interferon beta-IA, interferon gamma-IB, interferons, interleukins, iobenguane, iododoxorubicin, iproplatm, irinotecan, irinotecan hydrochloride, iroplact, irsogladine, isobengazole, isohomohalicondrin B, itasetron, jasplakinolide, kahalalide F, lamellarin-N triacetate, lanreotide, lanreotide acetate, leinamycin, lenograstim, lentinan sulfate, leptolstatin, letrozole, leukemia inhibiting factor, leukocyte alpha interferon, leuprolide acetate, leuprolide/estrogen/progesterone, leuprorelin, levamisole, liarozole, liarozole hydrochloride, linear polyamine analog, lipophilic disaccharide peptide, lipophilic platinum compounds, lissoclinamide, lobaplatin, lombricine, lometrexol, lometrexol sodium, lomustine, lonidamine, losoxantrone, losoxantrone hydrochloride, lovastatin, loxoribine, lurtotecan, lutetium texaphyrin lysofylline, lytic peptides, maitansine, mannostatin A, marimastat, masoprocol, maspin, matrilysin inhibitors, matrix metalloproteinase inhibitors, maytansine, mechlorethamine hydrochloride, megestrol acetate, melengestrol acetate, melphalan, menogaril, merbarone, mercaptopurine, meterelin, methioninase, methotrexate, methotrexate sodium, metoclopramide, metoprine, meturedepa, microalgal protein kinase C uihibitors, MIF inhibitor, mifepristone, miltefosine, mirimostim, mismatched double stranded RNA, mitindomide, mitocarcin, mitocromin, mitogillin, mitoguazone, mitolactol, mitomalcin, mitomycin, mitomycin analogs, mitonafide, mitosper, mitotane, mitotoxin fibroblast growth factor-saporin, mitoxantrone, mitoxantrone hydrochloride, mofarotene, molgramostim, monoclonal antibody, human chorionic gonadotrophin, monophosphoryl lipid a/myobacterium cell wall SK, mopidamol, multiple drug resistance gene inhibitor, multiple tumor suppressor 1-based therapy, mustard anticancer agent, mycaperoxide B, mycobacterial cell wall extract, mycophenolic acid, myriaporone, n-acetyldinaline, nafarelin, nagrestip, naloxone/pentazocine, napavin, naphterpin, nartograstim, nedaplatin, nemorubicin, neridronic acid, neutral endopeptidase, nilutamide, nisamycin, nitric oxide modulators, nitroxide antioxidant, nitrullyn, nocodazole, nogalamycin, n-substituted benzamides, O6-benzylguanine, octreotide, okicenone, oligonucleotides, onapristone, ondansetron, oracin, oral cytokine inducer, ormaplatin, osaterone, oxaliplatin, oxaunomycin, oxisuran, paclitaxel, paclitaxel analogs, paclitaxel derivatives, palauamine, palmitoylrhizoxin, pamidronic acid, panaxytriol, panomifene, parabactin, pazelliptine, pegaspargase, peldesine, peliomycin, pentamustine, pentosan polysulfate sodium, pentostatin, pentrozole, peplomycin sulfate, perflubron, perfosfamide, perillyl alcohol, phenazinomycin, phenylacetate, phosphatase inhibitors, picibanil, pilocarpine hydrochloride, pipobroman, piposulfan, pirarubicin, piritrexim, piroxantrone hydrochloride, placetin A, placetin B, plasminogen activator inhibitor, platinum complex, platinum compounds, platinum-triamine complex, plicamycin, plomestane, porfimer sodium, porfiromycin, prednimustine, procarbazine hydrochloride, propyl bis-acridone, prostaglandin J2, prostatic carcinoma antiandrogen, proteasome inhibitors, protein A-based immune modulator, protein kinase C inhibitor, protein tyrosine phosphatase inhibitors, purine nucleoside phosphorylase inhibitors, puromycin, puromycin hydrochloride, purpurins, pyrazorurin, pyrazoloacridine, pyridoxylated hemoglobin polyoxyethylene conjugate, RAF antagonists, raltitrexed, ramosetron, RAS farnesyl protein transferase inhibitors, RAS inhibitors, RAS-GAP inhibitor, retelliptine demethylated, rhenium RE 186 etidronate, rhizoxin, riboprine, ribozymes, RH retinarnide, RNAi, rogletimide, rohitukine, romurtide, roquinimex, rubiginone BI, ruboxyl, safingol, safingol hydrochloride, saintopin, sarcnu, sarcophytol A, sargramostim, SDI1 mimetics, semustine, senescence derived inhibitor 1, sense oligonucleotides, signal transduction inhibitors, signal transduction modulators, simtrazene, single chain antigen binding protein, sizofiran, sobuzoxane, sodium borocaptate, sodium phenylacetate, solverol, somatomedin binding protein, sonermin, sparfosafe sodium, sparfosic acid, sparsomycin, spicamycin D, spirogermanium hydrochloride, spiromustine, spiroplatin, splenopentin, spongistatin 1, squalamine, stem cell inhibitor, stem-cell division inhibitors, stipiamide, streptonigrin, streptozocin, stromelysin inhibitors, sulfinosine, sulofenur, superactive vasoactive intestinal peptide antagonist, suradista, suramin, swainsonine, synthetic glycosaminoglycans, talisomycin, tallimustine, tamoxifen methiodide, tauromustine, tazarotene, tecogalan sodium, tegafur, tellurapyrylium, telomerase inhibitors, teloxantrone hydrochloride, temoporfin, temozolomide, teniposide, teroxirone, testolactone, tetrachlorodecaoxide, tetrazomine, thaliblastine, thalidomide, thiamiprine, thiocoraline, thioguanine, thiotepa, thrombopoietin, thrombopoietin mimetic, thymalfasin, thymopoietin receptor agonist, thymotrinan, thyroid stimulating hormone, tiazofurin, tin ethyl etiopurpurin, tirapazamine, titanocene dichloride, topotecan hydrochloride, topsentin, toremifene, toremifene citrate, totipotent stem cell factor, translation inhibitors, trestolone acetate, tretinoin, triacetyluridine, triciribine, triciribine phosphate, trimetrexate, trimetrexate glucuronate, triptorelin, tropisetron, tubulozole hydrochloride, turosteride, tyrosine kinase inhibitors, tyrphostins, UBC inhibitors, ubenimex, uracil mustard, uredepa, urogenital sinus-derived growth inhibitory factor, urokinase receptor antagonists, vapreotide, variolin B, velaresol, veramine, verdins, verteporfin, vinblastine sulfate, vincristine sulfate, vindesine, vindesine sulfate, vinepidine sulfate, vinglycinate sulfate, vinleurosine sulfate, vinorelbine, vinorelbine tartrate, vinrosidine sulfate, vinxaltine, vinzolidine sulfate, vitaxin, vorozole, zanoterone, zeniplatin, zilascorb, zinostatin, zinostatin stimalamer, or zorubicin hydrochloride.

Especially well-suited are hydrophobic tumor signalling inhibitors such as sunitinib, trametinib, dabrafenib, sorafenib, bortezomib, talazoparib, osimertinib, gefininitib, afatinib, erlotinib, lapatinib, neratinib, dacomitinib, bosutinib, Dasatinib, imatinib, nilotinib, ponatinib, ibrutinib, cabozantinib, pazopanib, regorafenib, vemurafenib, rucaparib, olaparib, niraparib, selumetinib, entrectinib, idasanutlin, ipatasertib, lorlatinib, axitinib, glasdegib, gedatolisib, barasertib, encorafenib, binimetinib, cobimetinib, ruxolitinib, SAR405838, MI-773, AGM-232, APG-115, siremadlin, staurosporine, capivarsetib, uprosertib, GSK2110183, ipatasertib, miransertib, BAY1125976, ravoxertinib, ulixertinib, fimepinostat, vorinostat, mocetinostat, belinostat, entinostat, alpelisib, GSK343, and nedisertib.

Once the micellar composition has been prepared, it may be combined with pharmaceutical acceptable non-active ingredients to form a pharmaceutical composition, according to another aspect of the invention. As would be appreciated by one of skill in this art, the carriers may be chosen based on the route of administration as described below, the location of the target issue, the drug being delivered, the time course of delivery of the drug, etc.

**Methods of Treatment**

In an embodiment, targeted micelles or micellar compositions in accordance with aspects of the present invention are used to treat, alleviate, ameliorate, relieve, delay onset of, inhibit progression of, reduce severity of, and/or reduce incidence of one or more symptoms or features of a disease, disorder, and/or condition.

In an embodiment, the micelles or micellar compositions may be used to treat cancer and/or cancer cells. In certain embodiments, the micelles or micellar compositions may be used to treat any cancer, wherein dPGS binding molecules are expressed on the surface of cancer cells or in the tumor neovasculature in a subject in need thereof. Examples of the dPGS-related indication include, but are not limited to, non-small cell lung cancer, small-cell lung cancer, pancreatic cancer, colorectal carcinoma, and glioblastoma.

The term "cancer" includes pre-malignant as well as malignant cancers. Cancers include, but are not limited to, prostate, gastric cancer, colorectal cancer, skin cancer, e.g., melanomas or basal cell carcinomas, lung cancer, cancers of the head and neck, bronchus cancer, pancreatic cancer, urinary bladder cancer, brain or central nervous system cancer, peripheral nervous system cancer, esophageal cancer, cancer of the oral cavity or pharynx, liver cancer, kidney cancer, testicular cancer, biliary tract cancer, small bowel or appendix cancer, salivary gland cancer, thyroid gland cancer, adrenal gland cancer, osteosarcoma, chondrosarcoma, cancer of hematological tissues, and the like. "Cancer cells" can be in the form of a tumor, exist alone within a subject (e.g., leukemia cells), or be cell lines derived from a cancer.

Cancer can be associated with a variety of physical symptoms. Symptoms of cancer generally depend on the type and location of the tumor. For example, lung cancer can cause coughing, shortness of breath, and chest pain, while colon cancer often causes diarrhea, constipation, and blood in the stool. However, to give but a few examples, the following symptoms are often generally associated with many cancers: fever, chills, night sweats, cough, dyspnea, weight loss, loss of appetite, anorexia, nausea, vomiting, diarrhea, anemia, jaundice, hepatomegaly, hemoptysis, fatigue, malaise, cognitive dysfunction, depression, hormonal disturbances, neutropenia, pain, non-healing sores, enlarged lymph nodes, peripheral neuropathy, and sexual dysfunction.

In an aspect of the invention, a method for the treatment of cancer (e.g. prostate cancer) is provided. In some embodiments, the treatment of cancer comprises administering a therapeutically effective amount of the micelle or the micellar composition to a subject in need thereof, in such amounts and for such time as is necessary to achieve the desired result. In certain embodiments, a "therapeutically effective amount" of the micelle or the micellar composition is that amount effective for treating, alleviating, ameliorating, relieving, delaying onset of, inhibiting progression of, reducing severity of, and/or reducing incidence of one or more symptoms or features of cancer.

In an aspect of the invention, a method for administering the micelle or the micellar composition to a subject suffering from cancer (e.g. prostate cancer) is provided. In some embodiments, the micelle or the micellar composition is administered to a subject in such amounts and for such time as is necessary to achieve the desired result (i.e. treatment of cancer). Appropriate therapeutic protocols involve administering a therapeutically effective amount of the micelle or the micellar composition to a healthy individual (i.e., a subject who does not display any symptoms of cancer and/or who has not been diagnosed with cancer). For example, healthy individuals may be "immunized" with the micelle or the micellar composition prior to development of cancer and/or onset of symptoms of cancer; at risk individuals (e.g., patients who have a family history of cancer; patients carrying one or more genetic mutations associated with development of cancer; patients having a genetic polymorphism associated with development of cancer; patients infected by a virus associated with development of cancer; patients with habits and/or lifestyles associated with development of cancer; etc.) can be treated substantially contemporaneously with (e.g., within 48 hours, within 24 hours, or within 12 hours of) the onset of symptoms of cancer. Of course individuals known to have cancer may receive a treatment with the micelle or the micellar composition at any time.

In an embodiment, the micelle or the micellar composition can be used to inhibit the growth of cancer cells, e.g., cancer cells expressing dPGS binding molecules. As used herein, the term "inhibits growth of cancer cells" or "inhibiting growth of cancer cells" refers to any slowing of the rate of cancer cell proliferation and/or migration, arrest of cancer cell proliferation and/or migration, or killing of cancer cells, such that the rate of cancer cell growth is reduced in comparison with the observed or predicted rate of growth of an untreated control cancer cell. The term "inhibits growth" can also refer to a reduction in size or disappearance of a cancer cell or tumor, as well as to a reduction in its metastatic

potential. Preferably, such an inhibition at the cellular level may reduce the size, deter the growth, reduce the aggressiveness, or prevent or inhibit metastasis of a cancer in a patient. Those skilled in the art can readily determine, by any of a variety of suitable indicia, whether cancer cell growth is inhibited.

Inhibition of cancer cell growth may be evidenced, for example, by arrest of cancer cells in a particular phase of the cell cycle, e.g., arrest at the G2/M phase of the cell cycle Inhibition of cancer cell growth can also be evidenced by direct or indirect measurement of cancer cell or tumor size. In human cancer patients, such measurements generally are made using well known imaging methods such as magnetic resonance imaging, computerized axial tomography and X-rays. Cancer cell growth can also be determined indirectly, such as by determining the levels of circulating carcinoembryonic antigen, prostate specific antigen or other cancer-specific antigens that are correlated with cancer cell growth. Inhibition of cancer growth is also generally correlated with prolonged survival and/or increased health and well-being of the subject.

In an aspect, the present invention relates to a method for manufacturing an amphiphilic copolymer according to the preceding explanations, comprising the following steps:

In one step, a carboxylated polymer chosen from the group consisting of carboxylated polycaprolactone, a carboxylated polylactic acid polymer, and a carboxylated copolymer of lactic acid and glycolic acid is provided.

In another step, a polyglycerol derivative starting material is provided, the polyglycerol derivative starting material comprising a polyglycerol backbone and an alkyl residue bonded to the polyglycerol backbone, the alkyl residue having at least six consecutive methylene residues, wherein the polyglycerol derivative starting material further comprises a (terminal) thioamine residue being covalently bonded to the alkyl residue in a direct or indirect manner. "Bonding in a direct manner" means that the thioamine residue is directly bonded to alkyl residue. "Bonding in an indirect manner" means that the thioamine residue is bonded to the alkyl residue via a linker. The linker may itself be hydrocarbon residue.

In another step, the carboxylated polymer is conjugated to the polyglycerol derivative starting material by an amide coupling. For this purpose, the thioamine residue of the linker reacts with the carboxyl residue of the second block so that an amide bond is formed and a covalent bond between the first block and the second block via the linker is established.

In another step, at least some hydroxyl groups of the polyglycerol backbone are sulfated or sulfonated to obtain an amphiphilic copolymer according to the preceding explanations.

The described method steps need not to be performed in the indicated sequence, but can rather be carried out in any desired sequence being appropriate for synthesizing the amphiphilic copolymer.

In an embodiment, the provided carboxylated polymer is obtained by reacting a polymer chosen from the group consisting of polycaprolactone, a polylactic acid polymer, and a copolymer of lactic acid and glycolic acid with an organic acid anhydride. By this reaction, a carboxylic group is introduced into the polymer so that a carboxylated polymer is obtained.

In an embodiment, the provided polyglycerol derivative starting material is obtained by polymerizing an alkenol comprising at least six carbon atoms and glycidol to obtain a monofunctional polyglycerol allyl. Afterwards, a mercapto alkyl carboxylic acid is added. Then, a reaction between the monofunctional polyglycerol allyl and the mercapto alkyl carboxylic acid is allowed to obtain a carboxylated polyglycerol. Then, a thioamine is reacted with the carboxylated polyglycerol to obtain the polyglycerol derivative starting material. The reaction mentioned last results in introducing a thioamine residue into the polyglycerol derivative molecule via an amide bond to the linker.

In an embodiment, at least some of the hydroxyl groups of the polyglycerol backbone are substituted by sulfate groups. In doing so, the sulfation reagent is applied. The sulfation reagent is, in an embodiment, a sulfur trioxide base complex (e.g., $SO_3$-Pyridine, or $SO_3$-triethylamine). Sulfur trioxide pyridine complex is particularly appropriate. The sulfation can be carried out as generally known in the art. It takes place at the free hydroxyl groups of the polyglycerol backbone so that a sulfated dendritic polyglycerol results.

Appropriate reaction conditions for the sulfation step comprise a reaction temperature of 40 °C to 80 °C, in particular of 45 °C to 75 °C, in particular of 50 °C to 70 °C, in particular of 55 °C to 65 °C, in particular of 60 °C to 80 °C, and/or a reaction duration of 12 hours to 2 days, in particular of 1 day to 1.5 days. Particular appropriate reaction conditions are a reaction temperature of 55 °C to 65 °C (such as 60 °C) and a reaction duration of approximately one day. A sulfonation can be carried out under the same reaction conditions with an appropriate sulfonation reagent.

All embodiments described with respect to the amphiphilic copolymer can be combined in any desired way and can be transferred individually or in any desired combination to the described micelle, its uses, to the micellar composition and to the method for manufacturing the amphiphilic copolymer. Likewise, all embodiments described with respect to the micelle and its uses can be combined in any desired way and can be transferred individually or in any desired combination to the described amphiphilic copolymer, to the described micellar composition and to the described method for manufacturing an amphiphilic copolymer.

Furthermore, all embodiments and variants of the micellar composition can be combined in any desired way and can be transferred individually or in any desired combination to the amphiphilic copolymer, to the micelle, its uses and to the method for manufacturing an amphiphilic copolymer.

Finally, also all variants and embodiments of the described method for manufacturing an amphiphilic copolymer can be combined in any desired way and can be transferred individually or in any desired domination to the amphiphilic copolymer, to the micelle, its uses and to the micellar composition.

Further details of aspects of the present invention will be explained with respect to exemplary embodiments and accompanying Figures. In the Figures:

| | |
|---|---|
| Figure 1 | shows a general synthetic route for an amphiphilic copolymer; |
| Figure 2 | shows a synthetic route for manufacturing monofunctional dPG-SS-$NH_2$; |
| Figure 3 | shows an $^1$H NMR spectrum of dPG-ether-C11 -undecen measured in methanol-d4 and its corresponding structure; |
| Figure 4 | shows an IR spectrum of dPG-COOH and its structure; |
| Figure 5 | shows an $^1$H NMR spectrum of mdPG-COOH measured in methanol-d4; |
| Figure 6 | shows an $^1$H NMR spectrum of dPG-SS-$NH_2$ measured in methanol-d4; |
| Figure 7 | shows an 1H-NMR of PCL-COOH in $CDCl_3$; |
| Figure 8 | shows a synthetic route for the synthesis of dPGS-SS-PCL; |
| Figure 9A | shows an $^1$H-NMR spectrum of sedimentation (PCL-COOH) in $CDCl_3$; |
| Figure 9B | shows an $^1$H-NMR spectrum of supernatant (dPGS-SS-PCL) in DMF-d7:$D_2O$; |
| Figure 10 | shows a DLS spectrum of loaded/unloaded PCL-SS-dPGS micelles; |
| Figure 11A | shows a first set of DLS plots of Sunitinib-loaded PCL-SS-dPGS micelles directly after Sephadex and after resuspension from dry state; |
| Figure 11B | shows a second set of DLS plots of Sunitinib-loaded PCL-SS-dPGS micelles directly after Sephadex and after resuspension from dry state; |
| Figure 12 | shows a plot for CMC determination by light scattering intensity of SU-PCL-SS-dPGS and empty PCL-SS-dPGS micelles with varying concentration (dilution series); |
| Figure 13 | shows the results of a release study of sunitinib-loaded micelles in presence of 10 mM GSH in dialysis set up for 1 week, at 37 °C, and the result of a leaching study of sunitinib-loaded micelles in MilliQ at 37 °C; |
| Figure 14 | shows the results of a cell viability study of unloaded PCL-SS-dPGS micelles; |
| Figure 15A | shows the results of a cell viability study of free Sunitinib malate (hydrophilic); |
| Figure 15B | shows the results of a cell viability study of free Sunitinib (hydrophobic); |
| Figure 15C | shows the results of a cell viability study of PCL-SS-dPGS-micellar-encapsulated Sunitinib (hydrophobic); |
| Figure 16 | shows DLS plots of dPGS-SS-PCL micelles in PBS buffer and after incubation with 10 mM GSH; and |
| Figure 17 | shows DLS plots of dPGS-SS-PLGA micelles in PBS buffer and after incubation with 10 mM GSH. |

**Description of the synthesis and physicochemical properties**

The synthesis of the amphiphilic copolymer was performed using a reactive coupling strategy that forms amide bonds. In brief, the polymerized hydrophobic second block (PCL, PLGA, or PLA) is reacted with succinic acid anhydride to provide the corresponding acid (e.g. PCL-COOH).

The hydrophilic first block consists of an 11-undecenyl-polyglycerol that is linked to thiopropionic acid and cysteamine to provide monofunctional dPG-SS-$NH_2$. This reactive polymer was then conjugated to the hydrophobic second block (e.g. PCL-COOH) by an amide coupling. The final block copolymer was obtained after sulfation of the hydroxyl groups. This reaction scheme is depicted in Figure 1.

**Materials.** 3,6-Dimethyl-1,4-dioxane-2,5-dione (Lactide), 1,4-Dioxane-2,5-dione (Glycolide), 1-Oxa-2-oxocycloheptane (Caprolactone), N-Ethyl-N-(propan-2-yl)propan-2-amine (DIPEA), 1,8-Diazabicyclo[5.4.0]undec-7-ene (DBU), 1,3,4,6,7,8-Hexahydro-2H-pyrimido[1,2-a]pyrimidine (TBD), (Benzotriazol-1-yloxy)tripyrrolidinophosphonium hexafluorophosphate (PyBOP), Oxolane-2,5-dione (succinic anhydride) N,N'-Diisopropylcarbodiimide (DIC), N-Hydroxysuccinimide (NHS), sodium hydride (NaH), 1-Hydroxybenzotriazole (HOBt), 1-ethyl-3-(3-dimethylaminopropyl) carbodiimide (EDC), Mercaptopropionic acid, and Sulfur trioxide pyridine complex (SOs*Py) were purchased from sigma Aldrich. Glycidol (96 %, Acros) was dried over $CaH_2$ overnight and freshly distilled prior to use. N,N-dimethylformamide, (DMF, 99.8 %, extra dry, Acros), ethanol (99.8 %, extra dry), THF (99.8 %, extra dry) and DCM (99.8 %, extra dry) were purchased from Acros. Cystamine dihydrochloride (Cystamine·2HCl, > 98 %, Alfa Aesar), dry triethylamine ($Et_3N$, 99 %, Alfa Aesar), 11-Bromo-1-undecene (Alfa Aesar), dichloromethane, pyridine, toluene, ethanol, methanol and tetrahydrofuran were used as received. Dialysis was performed in benzoylated cellulose tubing purchased from Sigma-Aldrich (MWCO 2 kDa) and standard regenerated cellulose tubing purchased from Spectrumlab (MWCO 1 kDa, 2 kDa, and 3.5-5 kDa).

**Characterization.** $^1$HNMR spectra were recorded on a Bruker ECX 400 spectrometer operating at 400 MHz using Pyridine-d5, $CDCl_3$, DMF-d7, $CD_3OD$-d4, $D_2O$, or DMSO-d6 as a solvent. The chemical shifts were calibrated against residual solvent signal. The molecular weight and polydispersity of the polymers were determined by a Waters

1515 gel permeation chromatograph (GPC) instrument equipped with two linear PLgel columns (Mixed-C) following a guard column and a differential refractive-index detector. The measurements were performed using THF (hydrophobic segments) or water (hydrophilic segments) as the eluent at a flow rate of 1.0 mL $min^{-1}$ at 30 °C and a series of narrow polystyrene standards for the calibration of the columns. The size of micelles was determined using dynamic light scattering (DLS) at 25 °C using Zetasizer Nano-ZS (Malvern Instruments) equipped with a 633 nm He-Ne laser using back-scattering detection. Elemental analysis was performed with a VARIO EL III (Elementar). IR spectra were recorded with Nicolet AVATAR 320 FT-IR 5 SXC (Thermo Fisher Scientific, Waltham, MA, USA) with a DTGS detector from 4000 to 650 $cm^{-1}$. Sample measurement was done by dropping a solution of compound and letting the solvent evaporate for a few seconds.

**Synthesis of monofunctional dendritic polyglycerol (dPG-SS-$NH_2$)**

The hydrophilic first block consisting of an 11-undecenyl-polyglycerol linked to thiopropionic acid and cysteamine provides the dPG-SS-$NH_2$ and was synthesized in a two-step reaction protocol by starting with 10-undecen-1-ol. The simple approach realizes the development of redox-sensitive dendritic polyglycerols with a monofunctional group as terminal unit (cf. Figure 2). These monofunctional dendritic polyglycerols were used to couple different hydrophobic blocks via an amide coupling reaction. The 10-undecen-1-ol is used as inert starter for the anionic ring opening polymerization with glycidol to obtain monofunctional allylated polyglycerols, which provides a platform for a variety of functionalization reactions. Additionally, the unique branched architecture of the polyglycerol with the multivalent 1,2-diols of the terminal glycerol units can be further modified to generate a core-shell-type architecture (cf. references 1 and 2).

Here, the allyl group was further modified by a thiol-ene and amide coupling, to form monofunctional dPG-COOH and redox sensitive dPG-SS-$NH_2$ respectively. After coupling the monofunctional dendritic polyglycerol with the hydrophobic counterpart (e.g. PCL-SA), the 1,2-diols were sulfated to provide a copolymer with active targeting to inflammation-related tumor tissues.

In the first step, the 10-undecen-1-ol was polymerized by an anionic ring opening polymerization to monofunctional dPG-allyl. For this purpose, the 10-undecen-1-ol (20.66 g, 0.12 mol) was deprotonated by potassium methoxide (KOH 0.31 g, 5 mL MeOH; MeOK, 15 % deprotonation) and water was evaporated at 60 °C under vacuum. The synthesis reactor was heated to 100 °C and glycidol (200 g, 2.7 mol) was added over a period of 24h. The success of reaction was evaluated by $^1$H-NMR as shown in Figure 3. After 26 h, the reaction temperature was reduced to 75 °C and 600 mL of dry Dimethylformamide (DMF) was added. Subsequently mercaptopropionic acid (29.50 g, 278 mmol) and azobisisobutyro-nitrile (AIBN) (4.56 g, 27.8 mmol) was admitted to the reaction mixture. The *in-situ* Thiol-ene reaction to the monofunctional dPG-ether-COOH occurred within 4h. The crude product was precipitated in acetone and purified by TFF dialysis in a water/ethanol mixture 10:1 (MWCO: 1000 Da) for 3 d. The solvent was removed under reduced pressure to achieve the monofunctional dPG-COOH (150 g) as viscous yellow polymer. The polymer was analyzed via GPC and a number average molecular weight (Mn) of 3.85 kg $mol^{-1}$ was obtained. The ratio of the weight average molecular weight Mw to Mn (Mw/Mn) was 1.63. The characteristic absorbance bond of carbonyl group at 1715 $cm^{-1}$ in IR spectrum (cf. Figure 4) and the absence of the allyl peak in the $^1$H NMR proved the successful reaction of 3-Mercaptopropionic acid with the dPG-ether-C11-undecen as shown in Figure 5.

For analysis, a sample was taken after the first polymerization step, diluted with methanol and stirred over ion exchange resin (Dowex® Monosphere® 650C UPW) overnight. After filtering off the resin, the crude product was purified by dialysis in distilled water (MWCO: 1000 Da) for 3 d. The compound was obtained as a viscous yellow polymer after lyophilization.

To couple the monofunctional dPG-COOH with the hydrophobic second block (e.g. PCL-SA), it has to be modified via an amidation coupling with cystamine (second step). Therefore, monofunctional dPG-COOH (25 g, 6.58 mmol) was dissolved in 500 mL MES-buffer (pH 5.0, 50 mM) and NHS (3.79 g, 32.89 mmol) and EDC·HCl (6.31 g, 32.89 mmol) was added to the polymer solution, and the mixture was stirred for 30 min at room temperature (r.t.) to form the active ester. Separately, cystamine (7.41 g, 32.89 mmol) was dissolved in 1L PB-buffer (pH 7.4, 100 mM) and added to the reaction flak. The reaction was stirred for 16h at room temperature. The raw product was precipitated in acetone and purified by TFF dialysis in a water (MWCO: 1000 Da) for 3 d. Finally, the solvent was removed under reduced pressure to achieve the monofunctional dPG-SS-$NH_2$ (16.8 g). The corresponding structure and $^1$H-NMR are shown in Figure 6.

Overall, it took two reaction steps to achieve a monofunctional dendritic polyglycerol with a redox-sensitive dithiol group for the further coupling of various hydrophobic blocks. The initial reaction of the 10-undecen-1-ol is performed in a solvent free procedure in a bulk polymerization process followed by an *in-situ* modification to the monofunctional dPG-COOH. The second modification to the dPG-SS-$NH_2$ is performed in aqueous media.

**Synthesis of the Hydrophobic Second Block (PLA-COOH, PLGA-COOH, PCL-COOH)**

All three variants of the hydrophobic second block (i.e., a block containing polycaprolactone (PCL), poly(lactic acid) (PLA), or poly(lactic-co-glycolic acid) (PLGA)) are generally synthesized by ring opening polymerization of ε-caprolactone, lactide and glycolide at room temperature, using ethanol as initiator and DBU or TBD as catalyst. The reaction is performed in dry solvents (DCM, THF or toluene) at room temperature in several hours. The polymerization is quenched by addition of succinic anhydride. Thus, the reaction can be performed in only one day with one purification step leading to high yields. The solvent is evaporated by rotary evaporator and the crude polymer is dissolved in low amount of THF and precipitated three times in cold methanol to remove small oligomers and unreacted monomers. The solvent was evaporated, and the acid-functionalized polymer is obtained as a white powder after precipitation in cold methanol and drying under vacuum.

**Synthesis of random PLGA-COOH**

Lactide (12.00 g, 83.26 mmol) was placed in a flame-dried 200 mL Schlenk flask equipped with a magnetic stir bar and a rubber septum. Subsequently, lactide was dried while stirring in vacuum over 30 min. Then, dry DCM (80 mL) was added to dissolve lactide to obtain homogenous solution, that was degassed over 15 min. For initiation of reaction, ethanol (111 μL, 1.89 mmol) and DBU (282 μL, 1.89 mmol) were added to the lactide solution and the polymerization mixture was stirred at room temperature. As fast as possible, dried glycolide (3.2 g, 3.22 mmol) dissolved in degassed dry THF (0.5 M) was added under use of a syringe pump (rate 0.1 mL/min) to the polymerization mixture. After addition of glycolide, the reaction was further stirred for 30 min at room temperature. The reaction mixture was terminated by addition of succinic anhydride (757 mg, 7.57 mmol) dissolved in dry THF (0.5 M) and stirred overnight at room temperature. The reaction mixture was concentrated in vacuum with following precipitation in cold methanol for three times. The polymer was obtained as a white solid with a yield of 76 %. The polymer was analyzed via GPC and a Mn: 10.4 kg mol-1, Mw/Mn = 1.43 was obtained. $^1$H-NMR in $CDCl_3$ confirmed that polymer with molecular weight of 9922 g/mol was obtained.

**Synthesis of PLA-COOH**

Lactide (15.00 g, 104.07 mmol) was placed in a flame-dried 200 mL Schlenk flask equipped with a magnetic stir bar and a rubber septum. Subsequently, lactide was dried while stirring in vacuum over 30 min. Then, dry DCM (100 mL) was added to dissolve lactide to obtain homogenous solution, that was degassed over 15 min. For initiation of reaction, ethanol (113 μL, 1.93 mmol) and DBU (345 μL, 1.89 mmol) were added to the lactide solution and the polymerization mixture was stirred at room temperature. The reaction mixture was terminated by addition of succinic anhydride (757 mg, 7.57 mmol) dissolved in dry THF (0.5 M) and stirred overnight at room temperature. The reaction mixture was concentrated in vacuum with following precipitation in cold methanol for three times. The polymer was obtained as a white solid with a yield of 76 %. The polymer was analyzed via GPC and a Mn: 12.1 kg mol-1, Mw/Mn = 1.29 was obtained. $^1$H-NMR in $CDCl_3$ confirmed that polymer with molecular weight of 8736 g/mol was obtained.

**Synthesis of PCL-COOH**

Caprolactone (15.00 g, 13.89 mL, 131.42 mmol) was placed in a flame-dried 250 mL Schlenk flask equipped with a magnetic stir bar and a rubber septum. Then, toluene (100 mL) was added to dissolve caprolactone to obtain homogenous solution, that was degassed over 15 min. For initiation of reaction, ethanol (110 μL, 1.88 mmol) and TBD (523 mg, 3.75 mmol) were added to the caprolactone-solution and the polymerization mixture was stirred at room temperature for 5h. The reaction mixture was terminated by addition of succinic anhydride (751 mg, 7.51 mol) dissolved in dry THF and stirred overnight. The reaction mixture was concentrated in vacuum with following precipitation in cold methanol (centrifugation, 7000 rpm, 30 min, two times). The polymer was obtained as a white solid with a yield of 90 %. The polymer was analyzed via GPC and a Mn: 8.6 kg $mol^{-1}$, Mw/Mn = 1.22 was obtained. $^1$H-NMR in pyridine-d5 showed that polymer with molecular weight of 6175 g/mol was obtained. Figure 7 shows an $^1$H-NMR spectrum in $CDCl_3$ to prove the purity of polymer.

**Coupling of the monofunctional dPG-SS-$NH_2$ with Hydrophobic Block**

In a flame-dried Schlenk flask equipped with a rubber septum and a magnetic stir bar PCL-COOH (3 g, 0.484 mmol) and benzotriazol-1-yl-oxytripyrrolidinophosphonium hexafluorophosphate (PyBOP) (0.302 g, 0.581 mmol) were dissolved in dry DMF (20 mL). Subsequently, N,N-diisopropylethylamine (DIPEA) (0.17 mL, 0.968 mmol) was added and the solution was stirred for 2h at rt to active the acid. Then, dPG-SS-NH2 (1.16 g, 0.323 mmol) dissolved in dry

DMF (10 mL) was added dropwise to the solution and the reaction mixture was further stirred overnight. The success of amide coupling was checked using ninhydrin test and could be confirmed by absence of color formation indication full conversion of all free amines. The solution was then transferred in a dialysis tubing (MWCO: 1kDa) and first dialyzed against MeOH for one day and then dialyzed against water for several days under intensive changing of water to remove the organic solvents. The product was then freeze-dried using lyophilization. For a detailed overview, the reaction scheme of the amide coupling and sulfation to finally obtain dPGS-SS-PCL is shown in Figure 8. This reaction can be carried out in the same way if PLA or PGLA are present in the second block.

**Sulfation of the amphiphilic copolymer (dPG-SS-Hydrophobic Block)**

Sulfation of the amphiphilic copolymer was performed using a previously established protocol (cf. Reference 3). To a stirred solution of dPG-SS hydrophobic block in N,N-dimethylformamide (DMF), a solution of SO3/pyridine complex was added dropwise at 60 °C under argon atmosphere. After addition, the mixture was reacted for 2 h at 60 °C and 24 h at r.t. Then, the pH of the solution was adjusted to pH 8.0 by 1M NaOH solution. Distilled water was added, and the product was obtained after dialysis with a NaCl solution (MWCO = 2 kDa), using an ever-decreasing NaCl concentration, until the medium was changed with distilled water. The dialysis process was performed for 96 h. Then the amphiphilic copolymer and remained hydrophobic polymer was separated by sedimentation overnight, wherein the amphiphilic copolymer stays in solution (supernatant). The supernatant was collected and was dried. The precipitation was treated the same. The copolymer (e.g. dPGS-SS-PCL) was obtained after lyophilization. According to the calculations, the sulfur content of the dPGS-SS-PCL measured by elemental analysis showed almost complete sulfation of the hydroxyl groups ( %N: 1.18, %C: 24.16, %H: 5.69, %S: 13:98). The $^{1}$HNMR spectrum (cf. Figure 9B) of sulfated di-block copolymer shows the signals of the hydrophobic block (4.78; 2.43; 1.74; 1.50; 1.39 ppm) and the hydrophilic block (3.60-4.4 ppm). The appearance of new signals at 4.33-4.17 ppm which are assigned to the methylene protons adjacent to the sulfate groups proves the sulfation reaction. The $^{1}$H NMR spectra of the sedimentation containing the non-sulfated starting material (cf. Figure 9A) and the product of the coupling reaction (cf. Figure 9B) show the successful conjugation and formation of amphiphilic di-block copolymer. In summary, it was possible to synthesize an amphiphilic copolymer with a redox-sensitive moiety.

**Preparation of Micelles (dPGS-SS-Hydrophobic block)**

The micelles were prepared using an evaporation method. Briefly, the polymer was dissolved in a mixture of acetone, which is Class 3 solvent, and water, wherein the organic solvent hinders the formation of micelles. Then, the homogeneous solution of polymer or polymer/drug solution was dropwise added to a stirred solution of MilliQ. Subsequently, the organic solvent could be removed using a rotary evaporator.

**Preparation of micelles (c = 1 mg/mL)**

Micelles were prepared by dropwise addition of ultrasonicated acetone solution (100 μL) of dPGS-SS-PCL (1 mg, 10 mg mL$^{1}$) + 10 μL of MiliQ or PB (pH 7.4, 10 mM) to 990 μL MiliQ or phosphate buffer (PB, pH 7.4, 10 mM) under stirring (550 rpm) at r.t. for 1-2 min, followed by removal of acetone using rotary evaporation.

**Loading of the Drug (loading 20 wt%, c = 1 mg/mL).**

Drug-loaded micelles were prepared by dropwise addition of a mixed solution of copolymer (10 mg, Acetone: 1000 μL, 10 mg mL$^{-1}$) + 100 μL Mili-Q or PB and the drug (2 mg) 9900 μL MiliQ/PB (10 mM, pH 7.4) under stirring (550 rpm) at r.t. for 1-2 min, followed by removal of acetone using rotary evaporation. The drug-loaded micelle solution was passed over sephadex column (G25) to remove the non-encapsulated drug. Besides sephadex work up, the drug-loaded micelles can be purified by filtering the carrier with a syringe filter (200 nm regenerated cellulose, Sartorius). The drug loading efficiency and capacity was evaluated by UV-VIS measurement.

**Determination of Critical Micelle Concentration (CMC)**

The critical micelle concentration (CMC) of the loaded and unloaded micelles was determined by measuring the light scattering intensity with a Zetasizer. Briefly, the light scattering intensity of the micelles was measured at various concentrations (μg/mL) prepared in deionized water by serial dilution at 25 °C. By plotting the light scattering intensity against the log concentration of the carrier, the CMC was determined as the intersection of the best fit lines drawn through the data points.

**Drug Loading using UV**

The loaded micelles were dissolved in a mixture of methanol and Milli-Q. Then, the amount of Sunitinib in the carrier was determined by measuring the absorbance at 431 nm. Besides a calibration curve was prepared constructed with different concentrations of Sunitinib in methanol/Milli-Q. The drug loading content and drug loading efficiency were calculated according to the following formulas.

$$\boldsymbol{Drug\ loading\ content}\,(\%) = \frac{weight\ of\ Sunitinib\ in\ carrier}{total\ weight\ of\ Sunitinib\ in\ carrier} * 100\,\%$$

$$\boldsymbol{Drug\ loading\ effiency}\,(\%) = \frac{weight\ of\ Sunitinib\ in\ carrier}{weight\ of\ Sunitinib\ in\ feed} * 100\,\%$$

**Drug Leaching/Release study**

To evaluate the release/leaching of the described carrier system a dialysis setup was used. To hinder sink conditions a small volume of loaded micelles were dialyzed against big excess of dialysis medium (cut-off 3.5 kDa). The medium was MilliQ in case of leaching study. For release study to the medium 10 mM of GSH were added. Also, the MilliQ water was degassed to hinder further oxidation of GSH due to oxygen. The samples were placed on a shaking plate and were incubated for 7 days at 37 °C. At different time intervals the drug content in the sample was evaluated using the above described UV protocol.

**Physiochemical Characterization**

The synthesized micelles (empty, and drug loaded) were characterized by several techniques. Their size in solution was analyzed via dynamic light scattering (DLS). Here, native micelles and drug loaded micelles were analyzed in Milli-Q water. Interestingly, both, empty and loaded micelles showed similar size ranges of 52 nm (d.nm, intensity) with a defined polydispersity of 0.19 (cf. Table 1 and Figure 10). Furthermore, the surface charge of the micelles was measured by zeta potential measurements (ZP) and showed negatively charged particles (-66 mV, MilliQ-water) due to their sulfated groups at the micellar surface. Besides that, also loaded micelles showed a similar surface charge, and it can be assumed that the drug is located inside the hydrophobic parts of the micellar arrangement. By Cryo-TEM, slightly decreased micellar sizes were measured (21 nm) which can be explained by the methodical differences of the two measurements. In the Cryo-TEM, only the "naked" particles size was measured, whereas in the DLS measurements the hydrodynamic diameter is measured, which also takes the hydrate shell of the micelles into account and results in bigger diameter values. Micelles based on dPGS-SS-PLGA showed similar physiochemical properties.

**Table** 1: Characterization of unloaded dPGS-SS-PCL/PLGA micelles with DLS (c = 1 mg/mL).

| micelle | Molecular weight (g/mol) | Diameter [nm, intensity] | PDI | CMC (μg/mL) | CMC (mM) |
|---|---|---|---|---|---|
| dPGS-SS-PCL | 17000 | 52 | 0.19 | 5.4 | 3.18E-04 |
| dPGS-SS-PLGA | 17000 | 52 | 0.15 | 1.82 | 1.07E-04 |

**Drug Loading of the Micelles (CMC/DLC)/Cryo-Protection**

The micelles were loaded with the model drug sunitinib (20 wt%). Here, decent loading efficiencies of 13 wt% for dPGS-SS-PCL and 14.2 % for dPGS-SS-PLGA were observed. Interestingly, freeze dried loaded micelles (purified) showed similar physiochemical properties after resuspension in Milli-Q water, compared to the initial ones (52 nm, d.nm, intensity; 13 wt% DLC) (Figures 11A and 11B).

**Table 2:** Characterization of Sunitinib-loaded dPGS-SS-PCL/PLGA micelles with DLS.

| micelle | Diameter [nm, int.] | PDI | $DLC_{theo}$ [wt%] | $DLC_{UV}$ [wt%] | DLE [ %] | Leaching [ %][a] | Release, GSH [ %][b] | CMC (μg/mL) | CMC (mM) |
|---|---|---|---|---|---|---|---|---|---|
| dPGS-SS-PCL | 52.2 | 0.33 | 20 | 13 | 65 | below 1 | 99 | 0.50 | 2.94E-05 |
| dPGS-SS-PLGA | 59.2 | 0.25 | 20 | 14.2 | 71 | below 1 | 99 | 1.14 | 6.71E-05 |

[a] after 7 days, 37 °C, MilliQ.
[b] after 7 days, 37 °C, MilliQ, 10 mM GSH.

**Table 3:** Comparison of original/resuspended PCL-micelles in respect to obtained micellar size PDI.

| Sephadex | | Freeze-dried, filtered | |
|---|---|---|---|
| Size (nm, intensity) | PDI | Size (nm, intensity) | PDI |
| 52 | 0.31 | 96 | 0.24 |

In addition, the influence of encapsulated drug in respect to the CMC was investigated. Surprisingly, the CMC of loaded PCL-micelles ($2.94 \times 10^{-5}$ mM) decreased by factor 11 compared to the unloaded micelles, shown in Figure 12. The same phenomenon was observed for the PLGA analogues with factor 0.6. The low CMC values underline the high stability of the described micellar system. Interestingly, sunitinib stabilized the micellar system in the dry state via intermolecular interactions with the hydrophobic core, which allows a freeze drying/resuspension protocol without any cryo-protection agents.

In combination with the high loading values, the micelles were promising candidates as drug carrier system (DDS). The stability was further investigated by drug release/leaching studies in a physiological dialysis setup.

**Drug Release/Leaching**

The loaded micelles and their properties to stabilize and release the hydrophobic drug was analyzed in a dialysis setup. Here, no drug leaching was observed within 1 week for the dPGS-SS-PCL/PLGA micelles in aqueous media (Figure 13). However, in the presence of 10 mM glutathione (level in cytosol of cancerous cells) the disulfide linkage between the dPGS and the hydrophobic core units can be cleaved and triggers a micellar destabilization and associated drug release. The release study revealed a 50 % drug release for the dPGS-SS-PCL after 3.5 days, and 50 % drug releases for the PLGA analog after 2 days. The measurements of the dialysis setup confirm the findings from the physiochemical property measurements and underline the stability of the micellar system which can release the drug in a controlled manner und reductive environment. In comparison to that, micelles with a smaller molecular weight showed higher leaching and faster drug release characteristics.

**Cellular studies**

**Cell viability assay.**

HeLa cells were seeded in a transparent 96 well plate with a density of 10 000 cells per well and cultured for 24 hours. The medium (DMEM) was removed and replaced with medium containing PCL-SS-dPGS micelles (empty), followed by 48 hours of incubation. Subsequently, 10 μl of the pre-mixed Cell counting kit-8 (CCK-8) solution (Dojindo Molecular Technologies, Inc., Rockville, USA), containing the proprietary WST-8 tetrazolium salt, were added to each well. Viable cells reduce this salt to a formazan dye whose absorbance can be measured in the medium. Absorbance was measured at 450 nm using a Tecan Infinite 200 Pro microplate reader after two hours. Three independent experimental runs with triplicates each were performed (n = 3).

*In vitro* experiments were carried out to study the ability of the micelles to deliver drugs. In order to evaluate the optimal micellar concentration for the delivery of pharmaceutical active compounds, the highest non-toxic concentration was investigated using a CCK-8 cell viability assay during the period of 48 hours. The output of these measurements was that the empty carrier is non-toxic up to a concentration of 1.25 mg/mL (Figure 14). The IC-50 values of encapsulated

and free drug are in the same order of magnitude and are in good agreement to the stability, leaching and release studies (Figure 15A to 15C). Thus, the micelles appear to have high potency for the delivery of hydrophobic active drugs *in-vitro* and *in-vivo*.

In the following, additional information on the surface-potential and biodegradation of the micellar polymer systems of dPGS-SS-PCL and dPGS-SS-PLGA is provided. The formed micelles of dPGS-SS-PCL and dPGS-SS-PLGA were further characterized by their negative surface charge of the micellar outer identity and their responsiveness to GSH. As explained above, the polymer architecture comprises a hydrophobic segment, either PCL or PLGA, and a hydrophilic block, here dPGS, connected by a disulfide bridge (-S-S-) forming the amphiphilic copolymer. This structural segment is aimed to be cleaved under tumor conditions such as in the presence of GSH. Due to the sulfation ($-ROSO_3^- Na^+$) of the dendritic polyglycerol moieties (R-OH), the micelles possess a negative surface potential.

**Biodegradation of dPGS-SS-PCL and dPGS-SS-PLG by GSH**

The biodegradation of the dPGS micelles was studied by DLS and cryo-TEM measurements in more detail. The results are presented in Table 4. Upon incubation with 10 mM GSH, the PCL/PLGA-micelles changed their size, indicating that the disulfide bridge in the polymer structure got cleaved (Figures 16 and 17). The micelles formed by dPGS-SS-PCL revealed a size of 77 nm and micelles formed by dPGS-SS-PLGA of 131 nm in PBS, respectively. Upon the addition of reducing agents, the micelles shrank in their size, ending up in sizes of 52 nm and 117 nm for the PCL and PLGA amphiphilic copolymers.

**Table 4:** Characteristics of dPGS-SS-PCL and dPGS-SS-PLGA micelles regarding their size, biodegradation upon incubation with 10 mM GSH, and zeta-potential.

| micelle | Diameter [nm, int.][a] | Diameter (int. nm) 10 mM GSH, 24h[b] | Zeta-potential [MV][c] |
|---|---|---|---|
| dPGS-SS-PCL | 77 | 52 | -44.7 |
| dPGS-SS-PLGA | 131 | 117 | -43.0 |

[a]measured in PBS buffer at 25°C, pH 7.4
[b]measured in PBS buffer, pH 5.5
[c]measured in PB buffer at 25°C, pH 7.4

By evaluating cryo-TEM micrographs of dPGS-SS-PCL micelles in PBS and after 24h treatment with 10 mM GSH it could be shown that mot only a size shrinkage of the micelles occurs. Rather, the micelles fall apart when the disulfide bridge is cleaved by GSH. As observed by cryo-TEM, the micelles have a size of 60 nm in their native states, whereas the micelle size shrank to 40 nm in the presence of GSH. The TEM micrographs showed the spherical character of the formed micelles. In addition, the detected sizes by TEM matched the sizes determined by DLS.

Furthermore, a decreased number of particles was observed, indicating the disruption of micelles under reductive conductions supporting precise drug-release under tumor mirroring conditions. This observation matches with the previously performed release study where a promoted drug-release was detected in the presence of 10 mM GSH. Interestingly, several micelles were stable in the first 24h treatment with GSH. However, this result correlates with the sustainable release profile of the discussed drug delivery system. The cleavage of the disulfide bridge breaks down the micelles in a steady process leading to a prolonged drug release.

**Conclusion**

In summary, the additional measurements proof the anionic character of the present amphiphilic copolymers and their respective micelles as shown by the zeta potentials. Furthermore, biodegradation studies confirmed that GSH causes a disruption of the micelles, thus promoting a selectively drug-release at the desired site of action, e.g., cancerous tissue. The disulfide bridge in the polymer structure is sensitive against reducing agents.

**List of References**

(1) Haag, R.; Stumbé, J.-F.; Sunder, A.; Frey, H.; Hebel, A. Macromolecules 2000, 33, 8158.
(2) Wyszogrodzka, M.; Haag, R. Biomacromolecules 2009, 10, 1043.

(3) Bawa, K. K.; Jazani, A. M.; Shetty, C.; Oh, J. K. Macromolecular Rapid Communications 2018, 39, 1800477.
(4) Ferraro, M.; Silberreis, K.; Mohammadifar, E.; Neumann, F.; Dernedde, J.; Haag, R. Biomacromolecules 2018, 19, 4524.
(5) Skhiri, Y.; Gruner, P.; Semin, B.; Brosseau, Q.; Pekin, D.; Mazutis, L.; Goust, V.; Kleinschmidt, F.; El Harrak, A.; Hutchison, J. B.; Mayot, E.; Bartolo, J.-F.; Griffiths, A. D.; Taly, V.; Baret, J.-C. Soft Matter 2012, 8, 10618.
(6) Zhong Y, Dimde M, Stöbener D, Meng F, Deng C, Zhong Z, Haag R; ACS Appl Mater Interfaces 2016, 8, 27530.

**Claims**

1. Amphiphilic copolymer comprising a first block, a second block and a linker covalently linking the first block with the second block,

   wherein the first block is a hydrophilic dendritic polyglycerol derivative having a polyglycerol backbone and carrying a plurality of sulfate or sulfonate residues substituting hydroxyl groups of the polyglycerol backbone,
   wherein the second block is a hydrophobic block comprising a polymer chosen from the group consisting of polycaprolactone, a polylactic acid polymer, and a copolymer of lactic acid and glycolic acid,
   **characterized**
   **in that** the linker comprises a hydrocarbon having at least six consecutive methylene residues and a cleavable entity and in that the linker is devoid of a triazole-containing residue resulting from a reaction between an alkyne and an azide.

2. Amphiphilic copolymer according to claim 1, **characterized in that** the cleavable entity is a redox-sensitive entity or a pH-cleavable entity.

3. Amphiphilic copolymer according to claim 2, **characterized in that** the redox-sensitive entity is a disulfide bridge.

4. Amphiphilic copolymer according to claim 2, **characterized in that** the pH-cleavable entity is at least one an entity chosen from the group consisting of imines, oximes, hydrazones, and acetals.

5. Amphiphilic copolymer according to any of the preceding claims, **characterized in that** the polylactic acid polymer is chosen from the group consisting of poly-L-lactic acid, poly-D-lactic acid, and poly-D,L-lactic acid.

6. Amphiphilic copolymer according to any of the preceding claims, **characterized in that** the linker comprises 6 to 20 consecutive methylene residues.

7. Amphiphilic copolymer according to any of the preceding claims, **characterized in that** the polymer of the second block comprises 10 to 200 repeating units.

8. Amphiphilic copolymer according to any of the preceding claims, **characterized in that** the amphiphilic copolymer correspond to following general formula:

   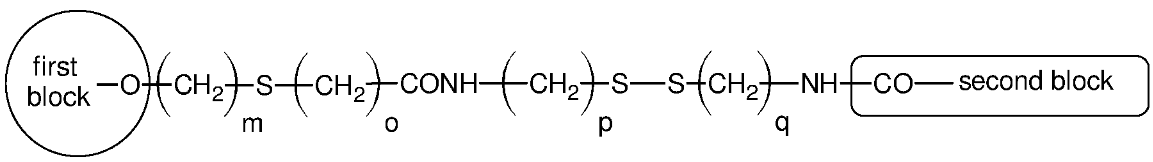

   wherein

   m = 6 to 20,
   o = 0 to 4,
   p = 0 to 4,
   q = 0 to 4.

9. Micelle, comprising a plurality of molecules of at least one amphiphilic copolymer according to any of the preceding claims.

**10.** Use of a micelle according to claim 9 for encapsulating an agent in an interior of the micelle.

**11.** Micellar composition, comprising a micelle according to claim 9 and an agent encapsulated in an interior of the micelle for use as a medicament.

**12.** Micellar composition, comprising a micelle according to claim 9 and an anti-tumor agent encapsulated in an interior of the micelle for use in treating a tumor.

**13.** Method for manufacturing an amphiphilic copolymer according to any of claims 1 to 8, comprising the following steps:

a) providing an carboxylated polymer chosen from the group consisting of carboxylated polycaprolactone, a carboxylated polylactic acid polymer, and a carboxylated copolymer of lactic acid and glycolic acid,
b) providing a polyglycerol derivative starting material comprising a polyglycerol backbone and an alkyl residue bonded to the polyglycerol backbone, the alkyl residue having at least six consecutive methylene residues, wherein the polyglycerol derivative starting material further comprises a thioamine residue being covalently bonded to the alkyl residue in a direct or indirect manner,
c) conjugating the carboxylated polymer to the polyglycerol derivative starting material by an amide coupling,
d) sulfating or sulfonating at least some hydroxyl groups of the polyglycerol backbone to obtain an amphiphilic copolymer according to any of claims 1 to 8.

**14.** Method according to claim 13, **characterized in that** the provided carboxylated polymer is obtained by reacting a polymer chosen from the group consisting of polycaprolactone, a polylactic acid polymer, and a copolymer of lactic acid and glycolic acid with an organic acid anhydride.

**15.** Method according to claim 13 or 14, **characterized in that** the provided polyglycerol derivative starting material is obtained by polymerizing an alkenol comprising at least six carbon atoms and glycidol to obtain a monofunctional polyglycerol allyl, adding a mercapto alkyl carboxylic acid and allowing a reaction between the monofunctional polyglycerol allyl and the mercapto alkyl carboxylic acid to obtain a carboxylated polyglycerol, reacting a thioamine with the carboxylated polyglycerol.

**Patentansprüche**

**1.** Amphiphiles Copolymer mit einem ersten Block, einem zweiten Block und einem Linker, der den ersten Block kovalent mit dem zweiten Block verbindet,

wobei der erste Block ein hydrophiles dendritisches Polyglycerinderivat mit einem Polyglycerin-Grundgerüst ist und eine Vielzahl von Sulfat- oder Sulfonatresten trägt, die Hydroxylgruppen des Polyglycerin-Grundgerüsts ersetzen,
wobei der zweite Block ein hydrophober Block ist, der ein Polymer umfasst, das aus der aus Polycaprolacton, einem Polymilchsäurepolymer und einem Copolymer von Milchsäure und Glycolsäure bestehenden Gruppe ausgewählt ist,
**dadurch gekennzeichnet,**
**dass** der Linker einen Kohlenwasserstoff mit mindestens sechs aufeinanderfolgenden Methylenresten und eine spaltbare Einheit umfasst und dass der Linker frei von einem triazolhaltigen Rest ist, der aus einer Reaktion zwischen einem Alkin und einem Azid resultiert.

**2.** Amphiphiles Copolymer nach Anspruch 1, **dadurch gekennzeichnet, dass** die spaltbare Einheit eine redoxempfindliche Einheit oder eine pH-spaltbare Einheit ist.

**3.** Amphiphiles Copolymer nach Anspruch 2, **dadurch gekennzeichnet, dass** die redoxempfindliche Einheit eine Disulfidbrücke ist.

**4.** Amphiphiles Copolymer nach Anspruch 2, **dadurch gekennzeichnet, dass** die pH-spaltbare Einheit mindestens eine Einheit ist, die aus der aus Iminen, Oximen, Hydrazonen und Acetalen bestehenden Gruppe ausgewählt ist.

**5.** Amphiphiles Copolymer nach einem der vorhergehenden Ansprüche, **dadurch gekennzeichnet, dass** das Polymilchsäurepolymer aus der aus Poly-L-Milchsäure, Poly-D-Milchsäure und Poly-D,L-Milchsäure bestehenden Gruppe ausgewählt ist.

6. Amphiphiles Copolymer nach einem der vorhergehenden Ansprüche, **dadurch gekennzeichnet, dass** der Linker 6 bis 20 aufeinanderfolgende Methylenreste umfasst.

7. Amphiphiles Copolymer nach einem der vorhergehenden Ansprüche, **dadurch gekennzeichnet, dass** das Polymer des zweiten Blocks 10 bis 200 sich wiederholende Einheiten umfasst.

8. Amphiphiles Copolymer nach einem der vorhergehenden Ansprüche, **dadurch gekennzeichnet, dass** das amphiphile Copolymer der folgenden allgemeinen Formel entspricht:

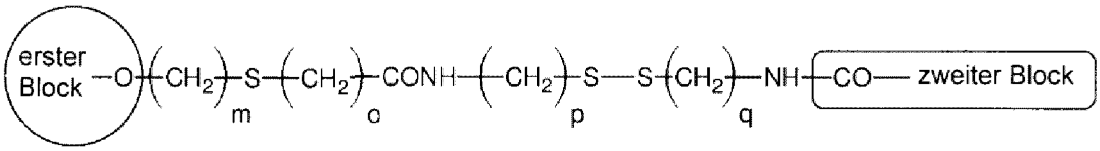

worin:

m = 6 bis 20,
o = 0 bis 4,
p = 0 bis 4,
q = 0 bis 4.

9. Mizelle, umfassend eine Vielzahl von Molekülen von mindestens einem amphiphilen Copolymer nach einem der vorhergehenden Ansprüche.

10. Verwendung einer Mizelle nach Anspruch 9 zum Einkapseln eines Wirkstoffs in einem Innenraum der Mizelle.

11. Mizellare Zusammensetzung, umfassend eine Mizelle nach Anspruch 9 und ein im Inneren der Mizelle eingekapseltes Mittel zur Verwendung als Medikament.

12. Mizellare Zusammensetzung, umfassend eine Mizelle nach Anspruch 9 und ein im Inneren der Mizelle eingekapseltes Anti-Tumor-Mittel zur Verwendung bei der Behandlung eines Tumors.

13. Verfahren zur Herstellung eines amphiphilen Copolymers nach einem der Ansprüche 1 bis 8, mit den folgenden Schritten:

a) Bereitstellen eines carboxylierten Polymers, das aus der aus carboxyliertem Polycaprolacton, einem carboxylierten Polymilchsäurepolymer und einem carboxylierten Copolymer von Milchsäure und Glycolsäure bestehenden Gruppe ausgewählt ist,
b) Bereitstellen eines Polyglycerinderivat-Ausgangsmaterials, das ein Polyglycerin-Grundgerüst und einen an das Polyglycerin-Grundgerüst gebundenen Alkylrest umfasst, wobei der Alkylrest mindestens sechs aufeinanderfolgende Methylenreste aufweist, wobei das Polyglycerinderivat-Ausgangsmaterial ferner einen Thioaminrest umfasst, der in direkter oder indirekter Weise kovalent an den Alkylrest gebunden ist,
c) Konjugieren des carboxylierten Polymers mit dem Polyglycerinderivat-Ausgangsmaterial durch eine Amidkopplung,
d) Sulfatieren oder Sulfonieren mindestens einiger Hydroxylgruppen des Polyglycerin-Grundgerüsts, um ein amphiphiles Copolymer nach einem der Ansprüche 1 bis 8 zu erhalten.

14. Verfahren nach Anspruch 13, **dadurch gekennzeichnet, dass** das bereitgestellte carboxylierte Polymer durch Umsetzung eines aus der aus Polycaprolacton, einem Polymilchsäurepolymer und einem Copolymer von Milchsäure und Glykolsäure bestehenden Gruppe ausgewählten Polymers mit einem organischen Säureanhydrid erhalten wird.

15. Verfahren nach Anspruch 13 oder 14, **dadurch gekennzeichnet, dass** das bereitgestellte Polyglycerinderivat-Ausgangsmaterial erhalten wird, indem ein Alkenol, das mindestens sechs Kohlenstoffatome umfasst, und Glycidol polymerisiert werden, um ein monofunktionelles Polyglycerinallyl zu erhalten, eine Mercaptoalkylcarbonsäure hinzugefügt wird und eine Reaktion zwischen dem monofunktionellen Polyglycerinallyl und der Mercaptoalkylcarbonsäure ermöglicht wird, um ein carboxyliertes Polyglycerin zu erhalten, und ein Thioamin mit dem carboxylierten Polyglycerin umgesetzt wird.

## Revendications

1. Copolymère amphiphile comprenant un premier bloc, un deuxième bloc et un segment de liaison liant de manière covalente le premier bloc au deuxième bloc,

   dans lequel le premier bloc est un dérivé dendritique hydrophile de polyglycérol ayant un squelette de polyglycérol et portant une pluralité de résidus sulfates ou sulfonates substituant les groupes hydroxyles du squelette de polyglycérol,
   dans lequel le deuxième bloc est un bloc hydrophobe comprenant un polymère choisi dans le groupe constitué par le polycaprolactone, un polymère d'acide polylactique et un copolymère d'acide lactique et d'acide glycolique,
   **caractérisé**
   **en ce que** le segment de liaison comprend un hydrocarbure ayant au moins six résidus méthylène consécutifs et une entité clivable et en ce que le segment de liaison est dépourvu d'un résidu contenant un triazole résultant d'une réaction entre un alcyne et un azoture.

2. Copolymère amphiphile selon la revendication 1, **caractérisé en ce que** l'entité clivable est une entité sensible à l'oxydoréduction ou une entité clivable au pH.

3. Copolymère amphiphile selon la revendication 2, **caractérisé en ce que** l'entité sensible à l'oxydoréduction est un pont disulfure.

4. Copolymère amphiphile selon la revendication 2, **caractérisé en ce que** l'entité clivable au pH est au moins une entité choisie dans le groupe constitué par les imines, les oximes, les hydrazones et les acétals.

5. Copolymère amphiphile selon l'une quelconque des revendications précédentes, **caractérisé en ce que** le polymère d'acide polylactique est choisi dans le groupe constitué par l'acide poly-L-lactique, l'acide poly-D-lactique et l'acide poly-D,L-lactique.

6. Copolymère amphiphile selon l'une quelconque des revendications précédentes, **caractérisé en ce que** le segment de liaison comprend de 6 à 20 résidus méthylène consécutifs.

7. Copolymère amphiphile selon l'une quelconque des revendications précédentes, **caractérisé en ce que** le polymère du deuxième bloc comprend 10 à 200 unités répétitives.

8. Copolymère amphiphile selon l'une quelconque des revendications précédentes, **caractérisé en ce que** le copolymère amphiphile correspond à la formule générale suivante :

   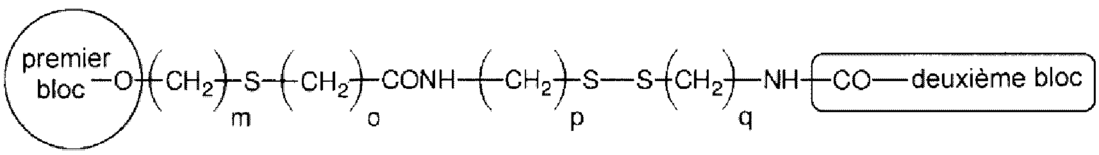

   où

   m = 6 à 20,
   o = 0 à 4,
   p = 0 à 4,
   q = 0 à 4.

9. Micelle, comprenant plusieurs molécules d'au moins un copolymère amphiphile selon l'une quelconque des revendications précédentes.

10. Utilisation d'une micelle selon la revendication 9 pour encapsuler un agent à l'intérieur de la micelle.

**11.** Composition micellaire, comprenant une micelle selon la revendication 9 et un agent encapsulé à l'intérieur de la micelle pour utilisation comme médicament.

**12.** Composition micellaire, comprenant une micelle selon la revendication 9 et un agent anti-tumoral encapsulé à l'intérieur de la micelle pour utilisation dans le traitement d'une tumeur.

**13.** Procédé pour la fabrication d'un copolymère amphiphile selon l'une quelconque des revendications 1 à 8, comprenant les étapes suivantes :

a) fournir un polymère carboxylé choisi dans le groupe constitué par le polycaprolactone carboxylé, un polymère d'acide polylactique carboxylé et un copolymère d'acide lactique et d'acide glycolique carboxylé,
b) fournir un matériau de départ dérivé du polyglycérol comprenant un squelette de polyglycérol et un résidu alkyle lié au squelette de polyglycérol, le résidu alkyle ayant au moins six résidus méthylène consécutifs, le matériau de départ dérivé du polyglycérol comprenant en outre un résidu thioamine lié de manière covalente au résidu alkyle d'une manière directe ou indirecte,
c) conjuguer le polymère carboxylé au matériau de départ dérivé du polyglycérol par un couplage amide,
d) sulfater ou sulfoner au moins certains groupes hydroxyles du squelette de polyglycérol pour obtenir un copolymère amphiphile selon l'une quelconque des revendications 1 à 8.

**14.** Procédé selon la revendication 13, **caractérisé en ce que** le polymère carboxylé fourni est obtenu en faisant réagir un polymère choisi dans le groupe constitué par le polycaprolactone, un polymère d'acide polylactique et un copolymère d'acide lactique et d'acide glycolique avec un anhydride d'acide organique.

**15.** Procédé selon la revendication 13 ou 14, **caractérisé en ce que** le matériau de départ dérivé du polyglycérol fourni est obtenu en polymérisant un alcénol comprenant au moins six atomes de carbone et du glycidol pour obtenir un allyle de polyglycérol monofonctionnel, en ajoutant un acide mercapto alkyl carboxylique et en permettant une réaction entre l'allyle de polyglycérol monofonctionnel et l'acide mercapto alkyl carboxylique pour obtenir un polyglycérol carboxylé, en faisant réagir une thioamine avec le polyglycérol carboxylé.

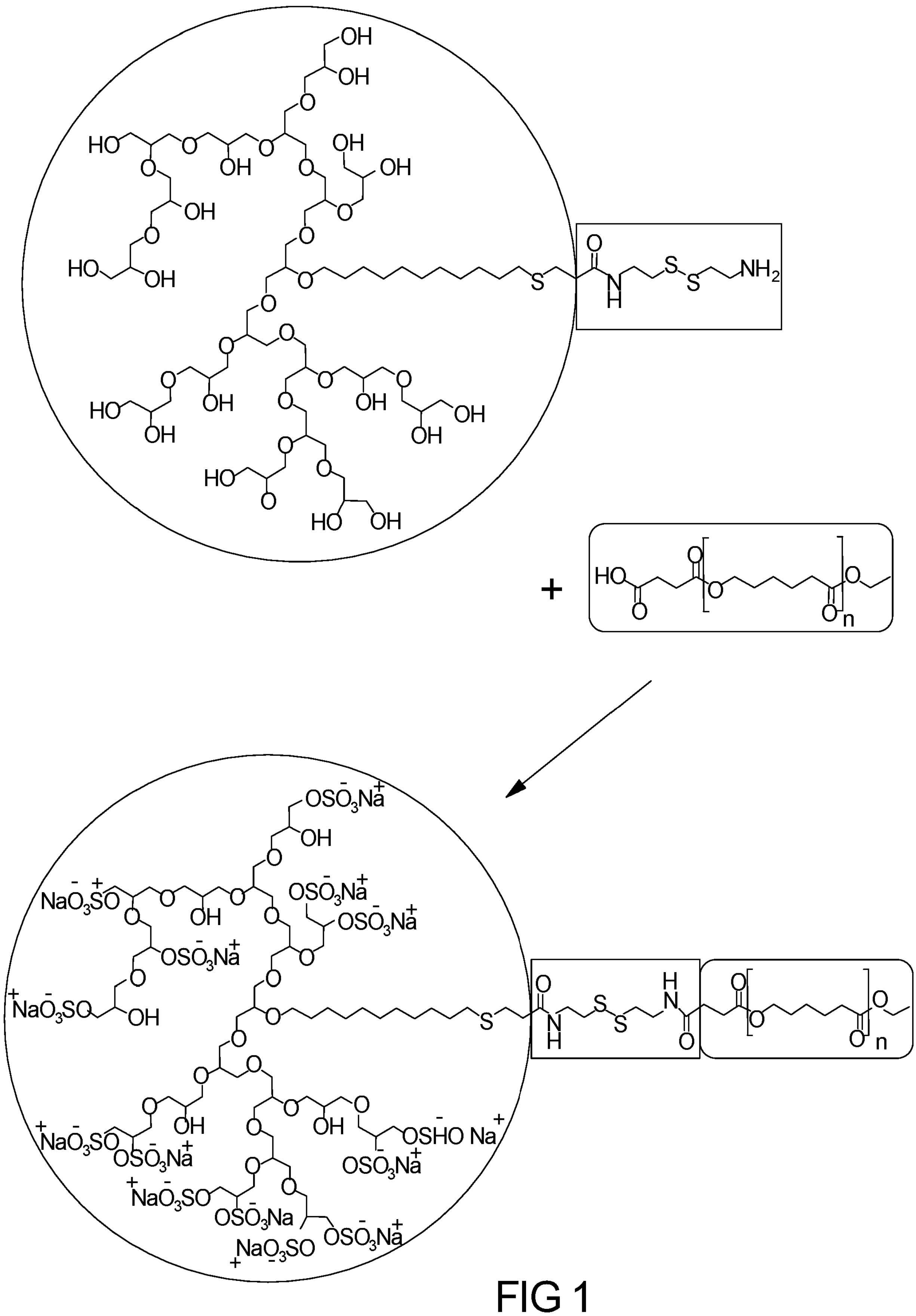

FIG 1

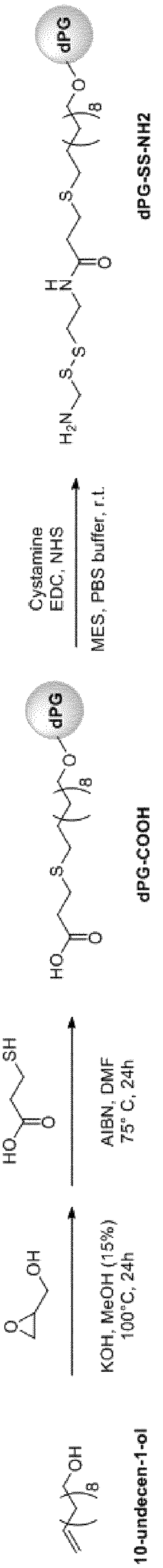
10-undecen-1-ol
KOH, MeOH (15%)
100°C, 24h
AIBN, DMF
75° C, 24h
dPG-COOH
Cystamine
EDC, NHS
MES, PBS buffer, r.t.
dPG-SS-NH2

FIG 2

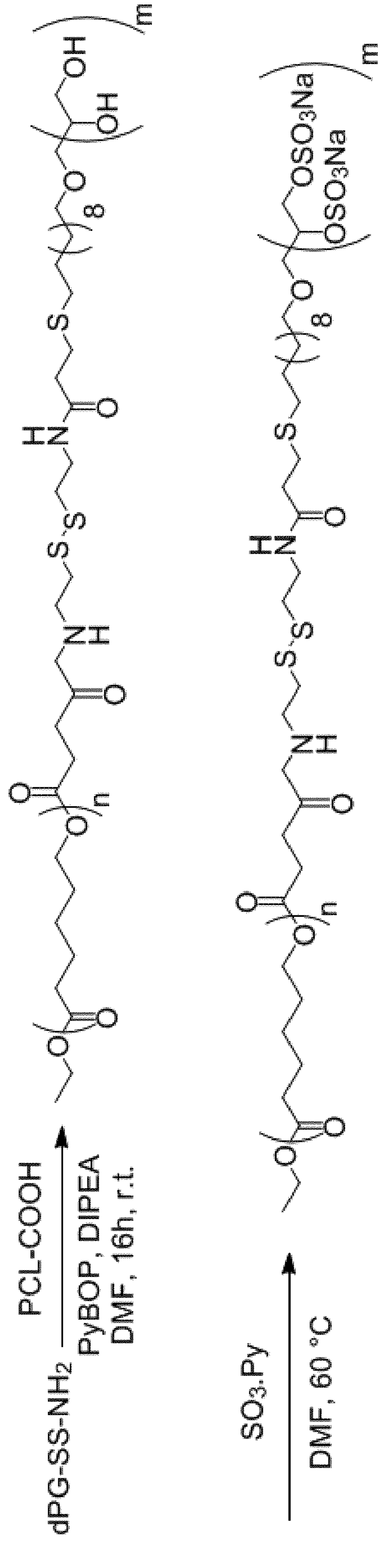
dPG-SS-NH$_2$
PCL-COOH
PyBOP, DIPEA
DMF, 16h, r.t.
SO$_3$.Py
DMF, 60 °C

FIG 8

FIG 3

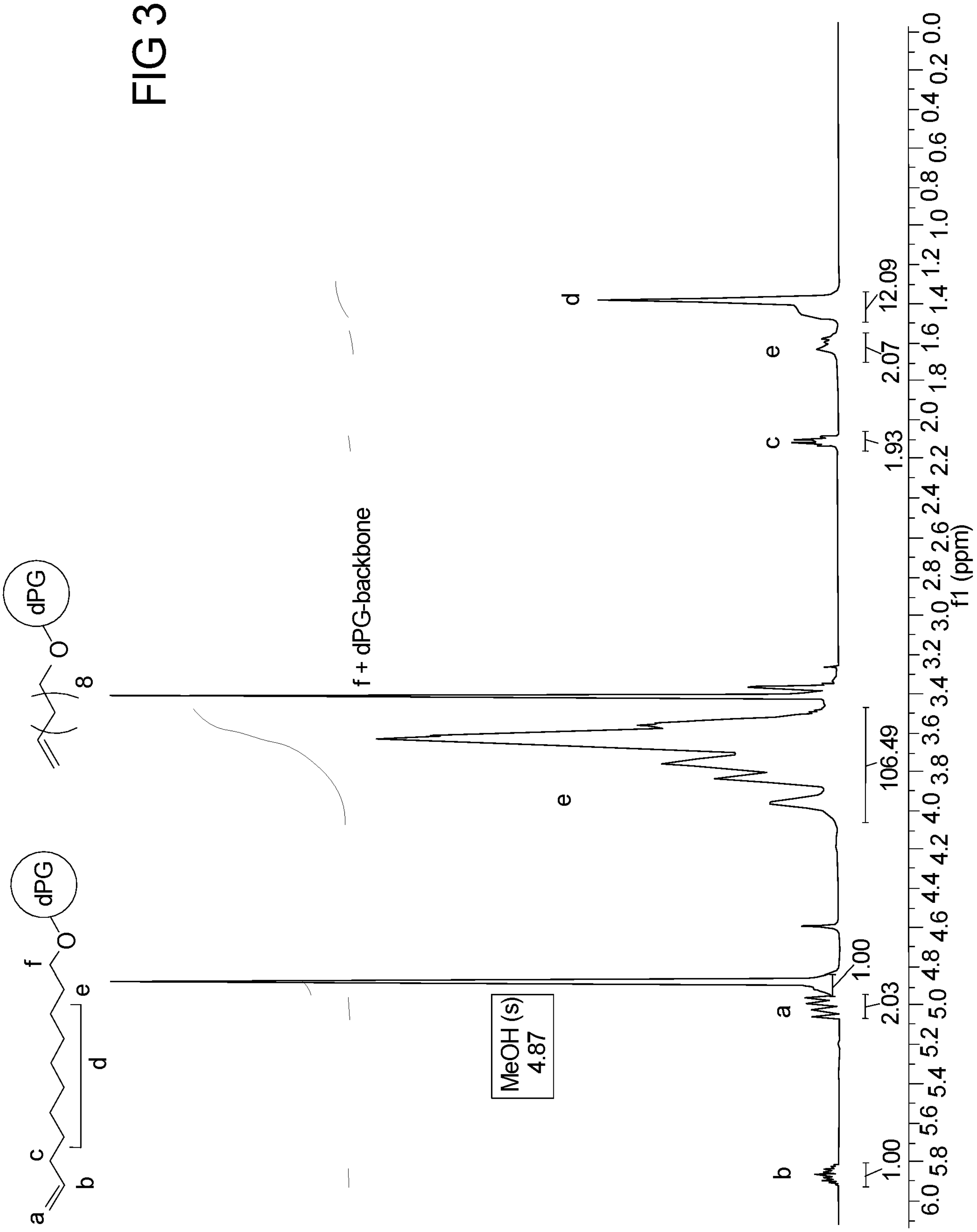
a
b
c
d
e
f
dPG
O
8
f + dPG-backbone
MeOH (s)
4.87
1.00
2.03
1.00
106.49
1.93
2.07
12.09
f1 (ppm)
6.0 5.8 5.6 5.4 5.2 5.0 4.8 4.6 4.4 4.2 4.0 3.8 3.6 3.4 3.2 3.0 2.8 2.6 2.4 2.2 2.0 1.8 1.6 1.4 1.2 1.0 0.8 0.6 0.4 0.2 0.0

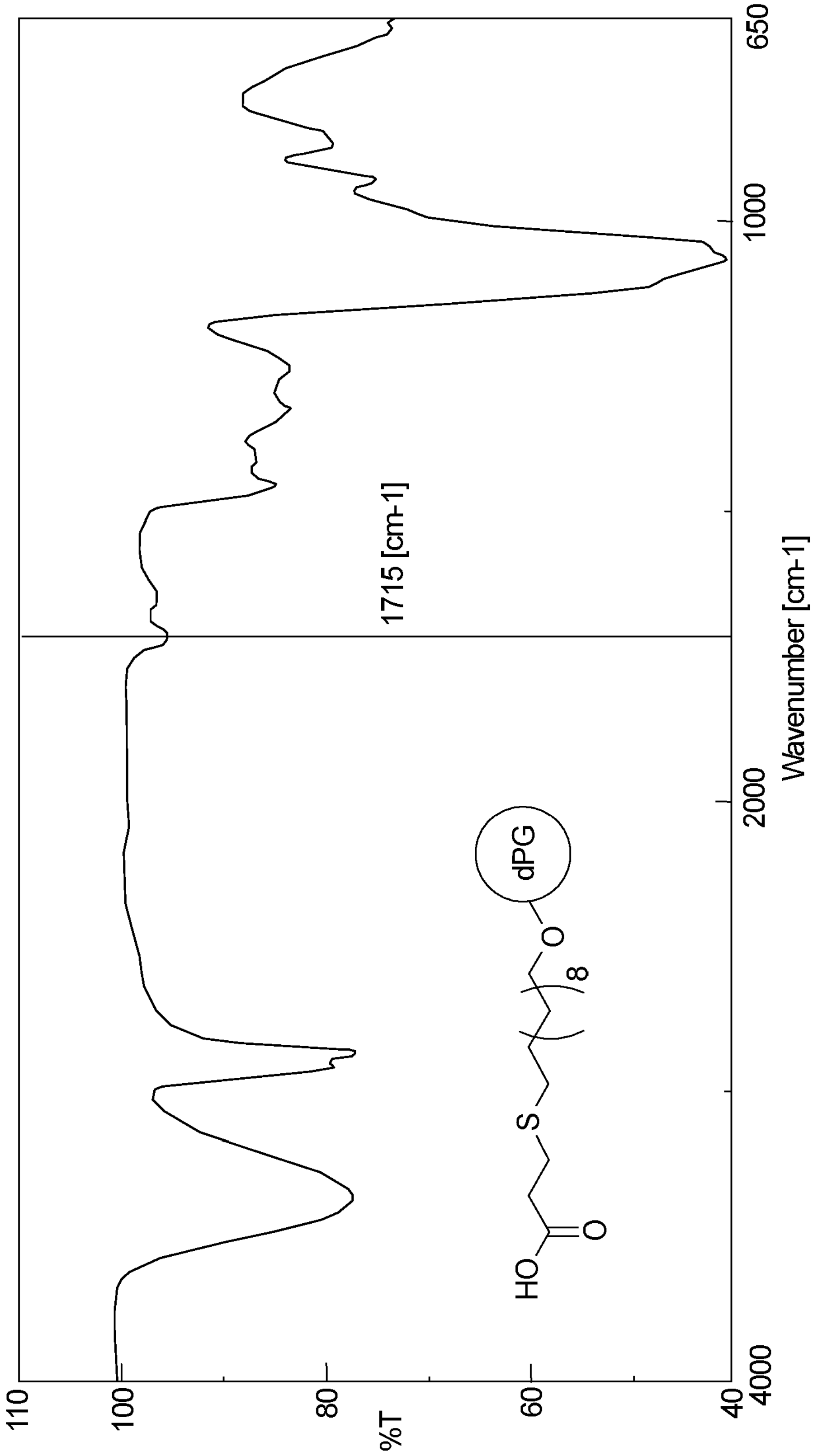


FIG 4

FIG 5

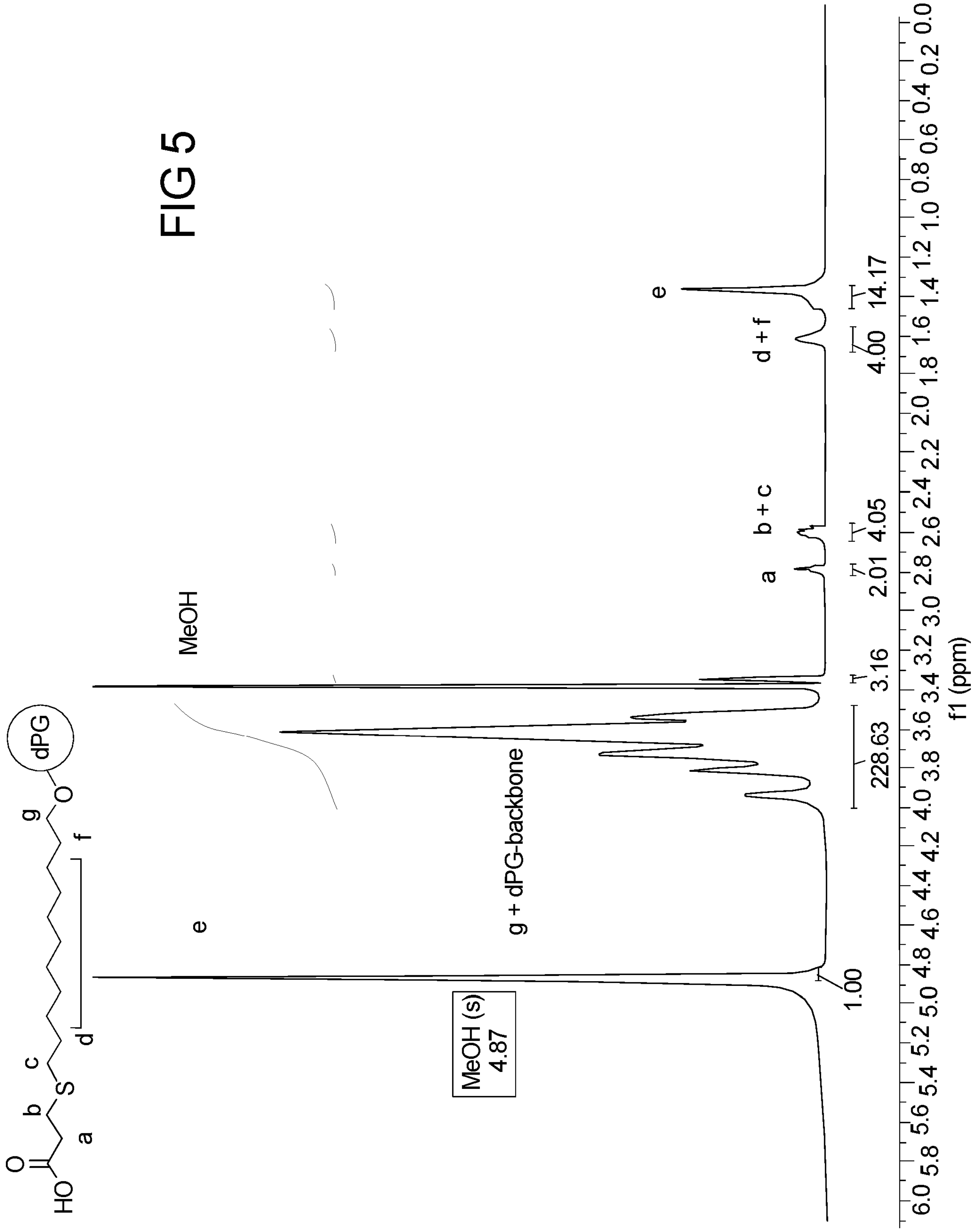
MeOH
e
d + f
b + c
a
g + dPG-backbone
MeOH (s)
4.87
14.17
4.00
4.05
2.01
3.16
228.63
1.00
6.0 5.8 5.6 5.4 5.2 5.0 4.8 4.6 4.4 4.2 4.0 3.8 3.6 3.4 3.2 3.0 2.8 2.6 2.4 2.2 2.0 1.8 1.6 1.4 1.2 1.0 0.8 0.6 0.4 0.2 0.0
f1 (ppm)

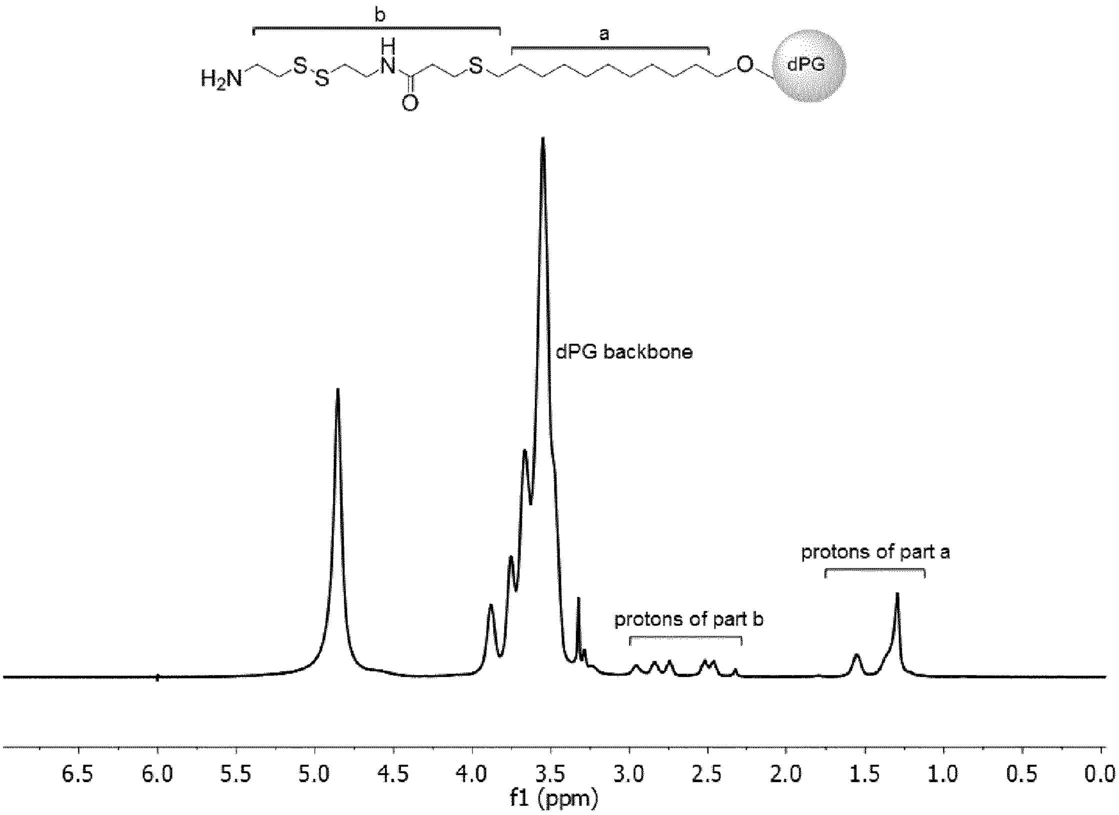
dPG backbone
protons of part a
protons of part b
6.5 6.0 5.5 5.0 4.5 4.0 3.5 3.0 2.5 2.0 1.5 1.0 0.5 0.0
f1 (ppm)

FIG 6

FIG 7

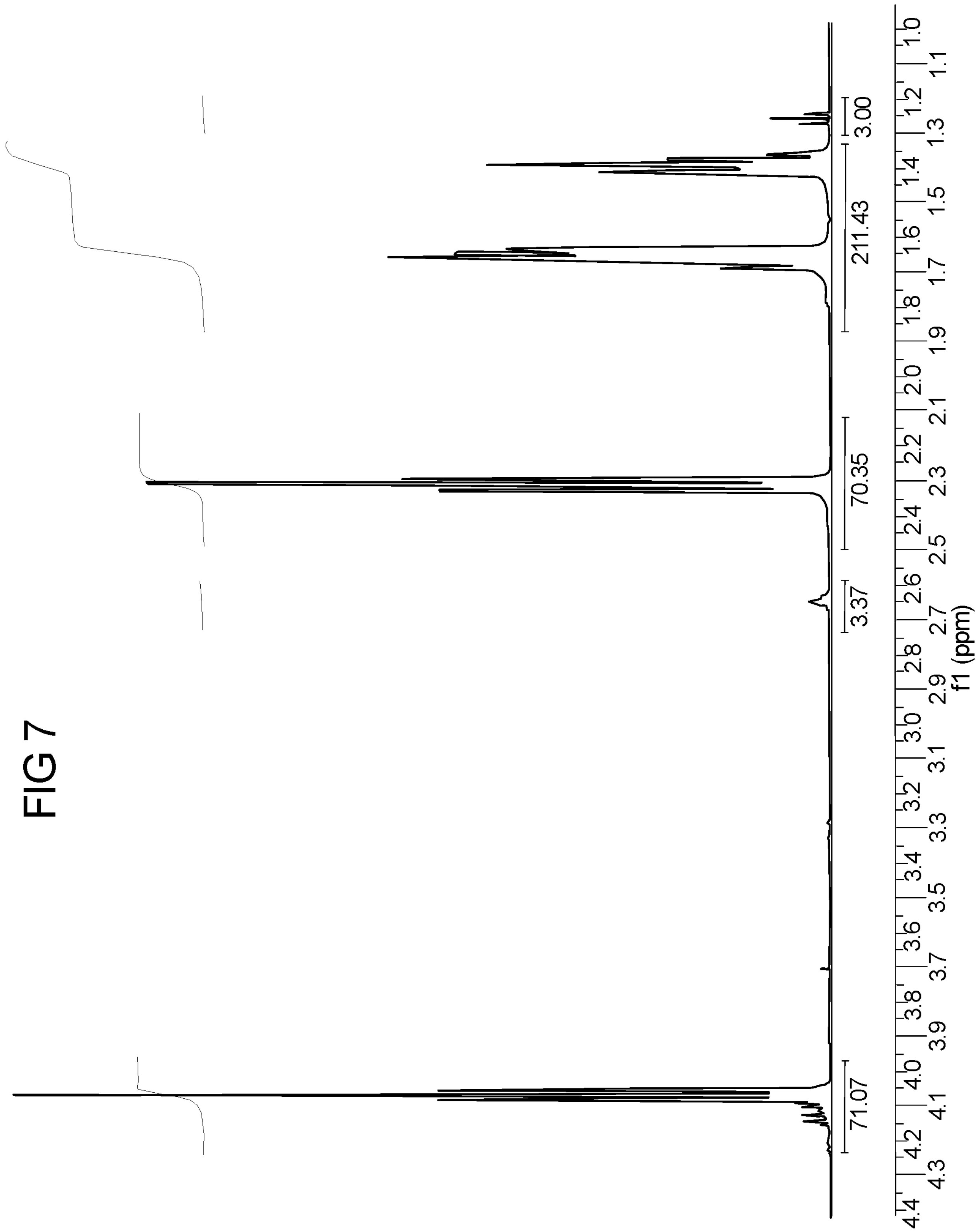
3.00
211.43
70.35
3.37
71.07
4.4 4.3 4.2 4.1 4.0 3.9 3.8 3.7 3.6 3.5 3.4 3.3 3.2 3.1 3.0 2.9 2.8 2.7 2.6 2.5 2.4 2.3 2.2 2.1 2.0 1.9 1.8 1.7 1.6 1.5 1.4 1.3 1.2 1.1 1.0
f1 (ppm)

FIG 9A

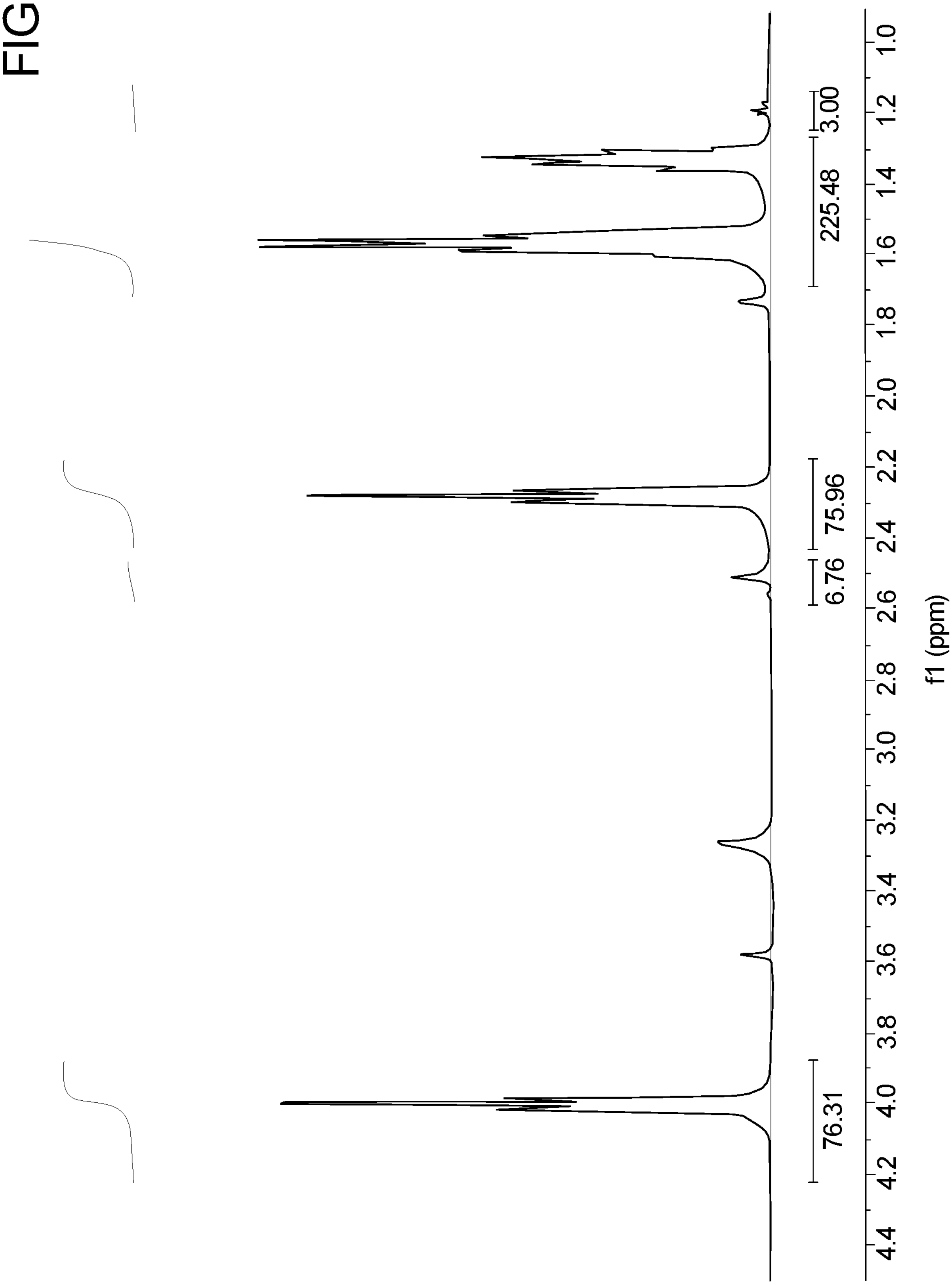
3.00
225.48
75.96
6.76
76.31
1.0
1.2
1.4
1.6
1.8
2.0
2.2
2.4
2.6
2.8
3.0
3.2
3.4
3.6
3.8
4.0
4.2
4.4
f1 (ppm)

FIG 9B

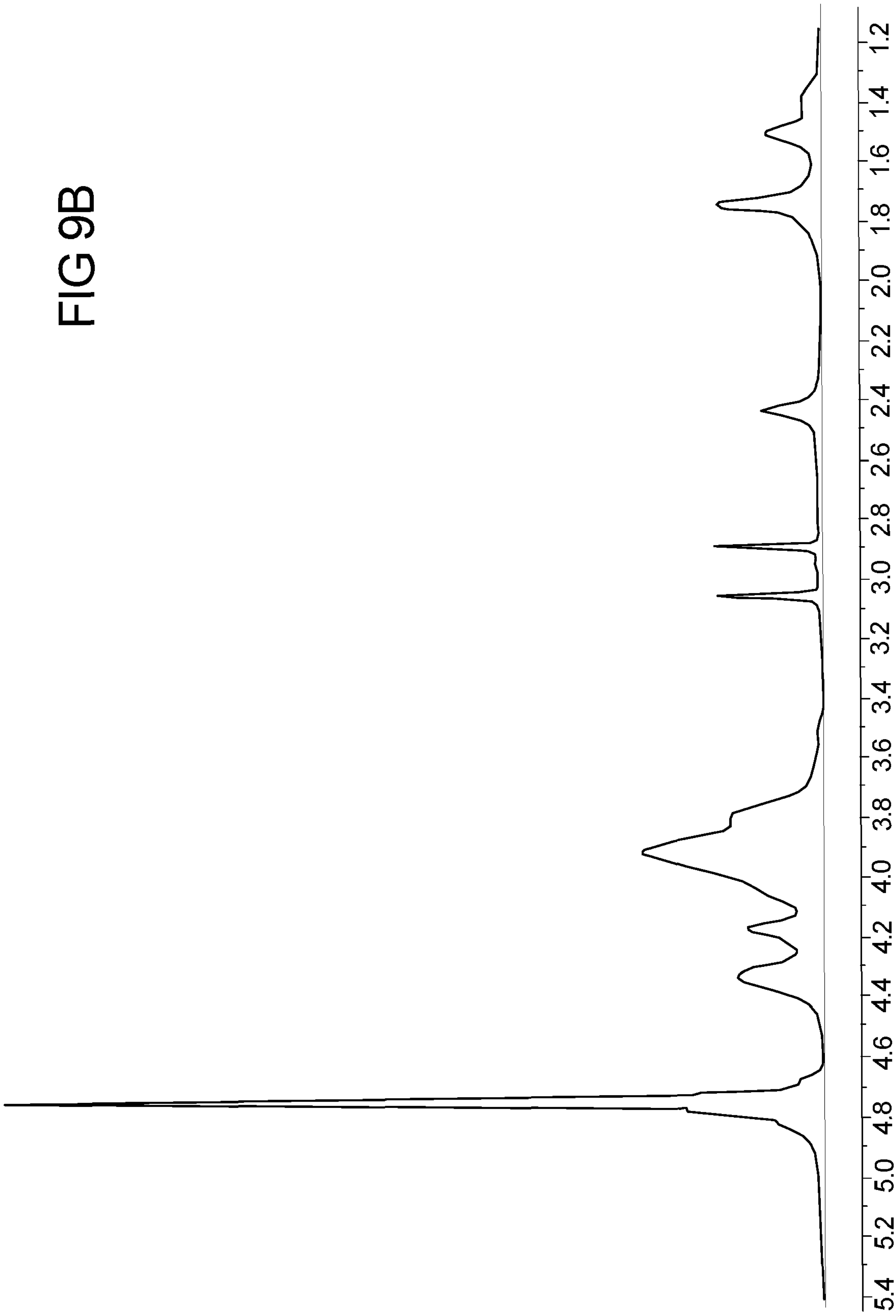

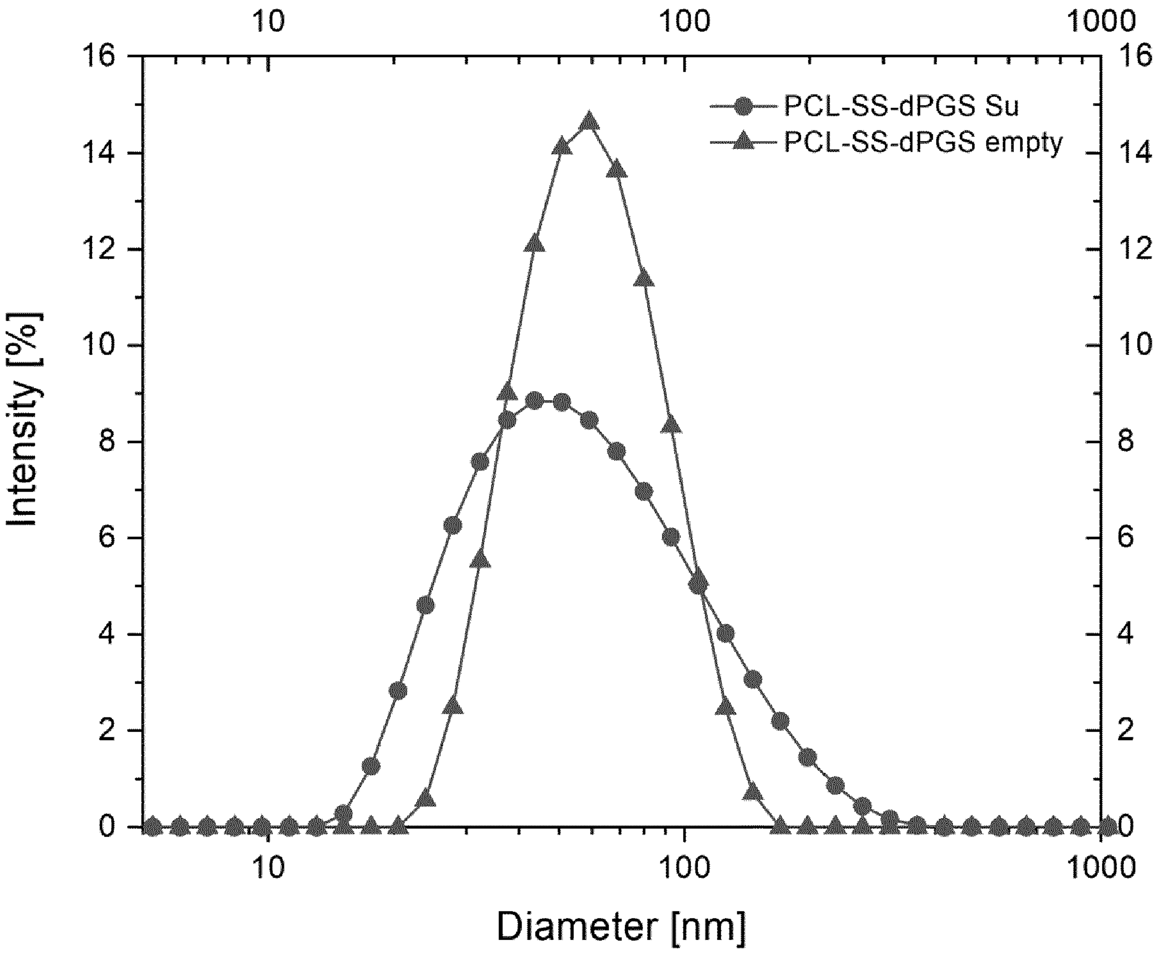
10
100
1000
16
14
12
10
8
6
4
2
0
Intensity [%]
PCL-SS-dPGS Su
PCL-SS-dPGS empty
Diameter [nm]

FIG 10

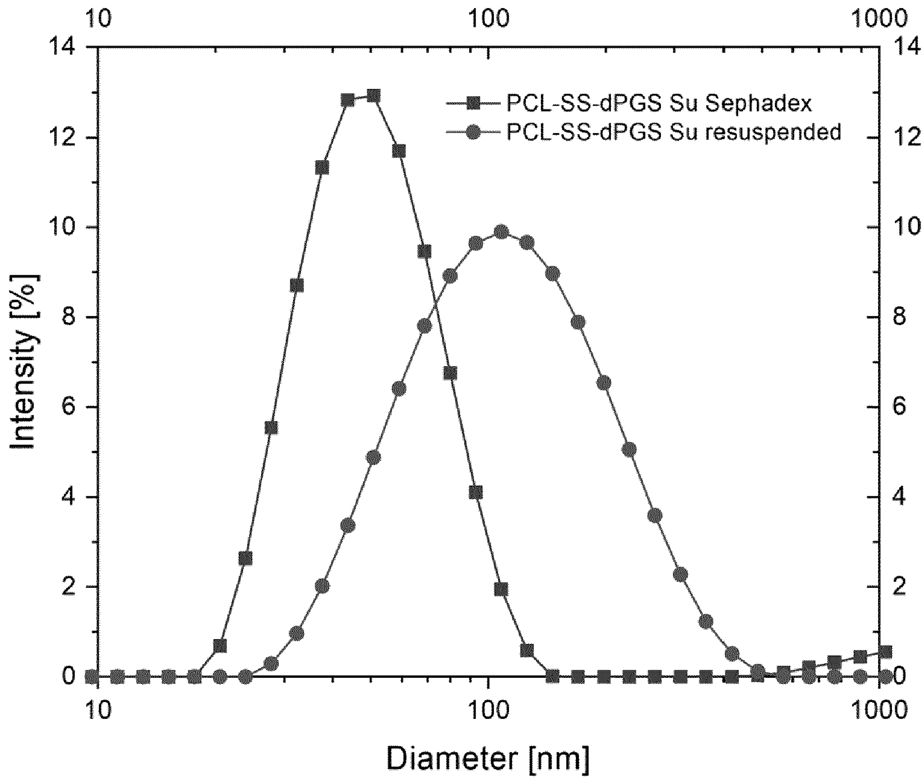


FIG 11A

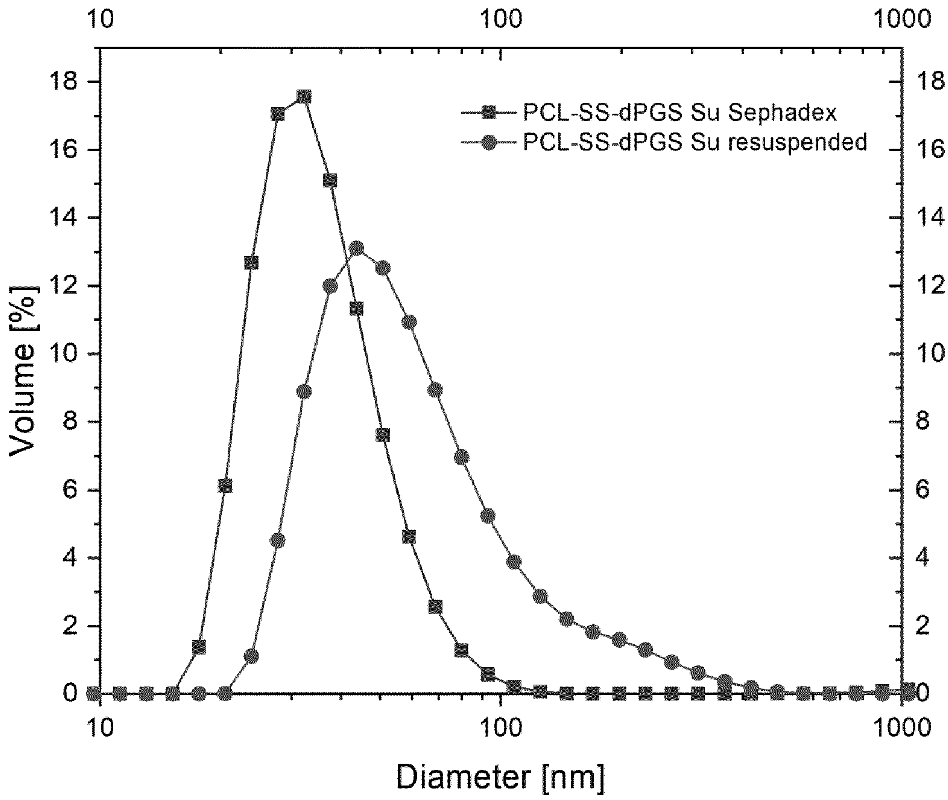


FIG 11B

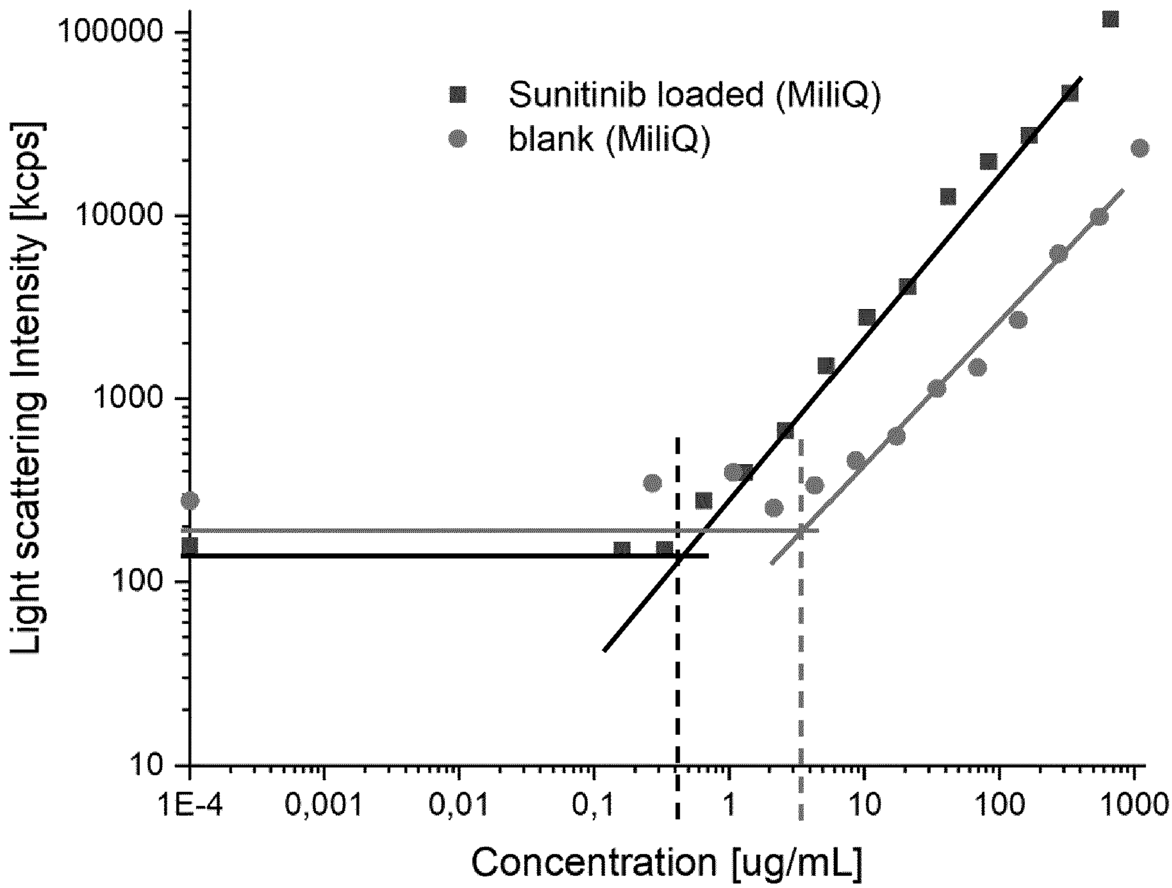
100000
10000
1000
100
10
Light scattering Intensity [kcps]
Sunitinib loaded (MiliQ)
blank (MiliQ)
1E-4
0,001
0,01
0,1
1
10
100
1000
Concentration [ug/mL]

FIG 12

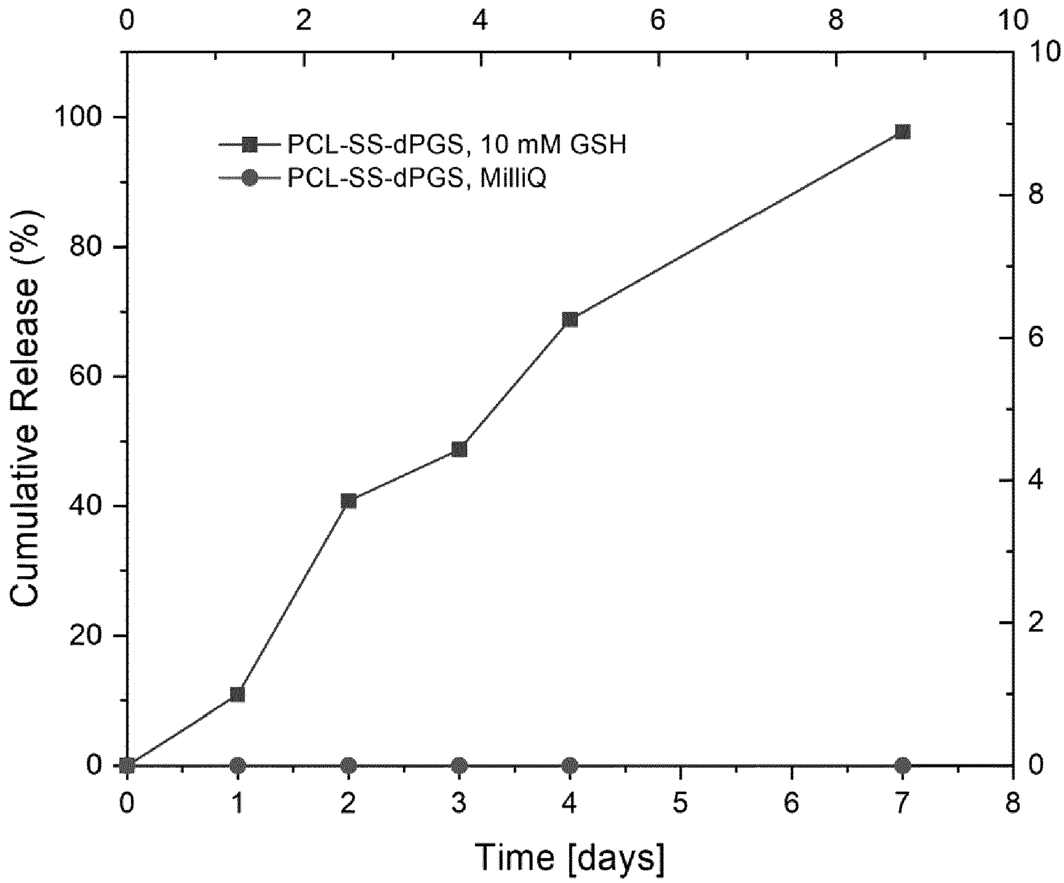
0
2
4
6
8
10
10
100
8
80
6
60
4
40
2
20
0
0
0
1
2
3
4
5
6
7
8
PCL-SS-dPGS, 10 mM GSH
PCL-SS-dPGS, MilliQ
Cumulative Release (%)
Time [days]

FIG 13

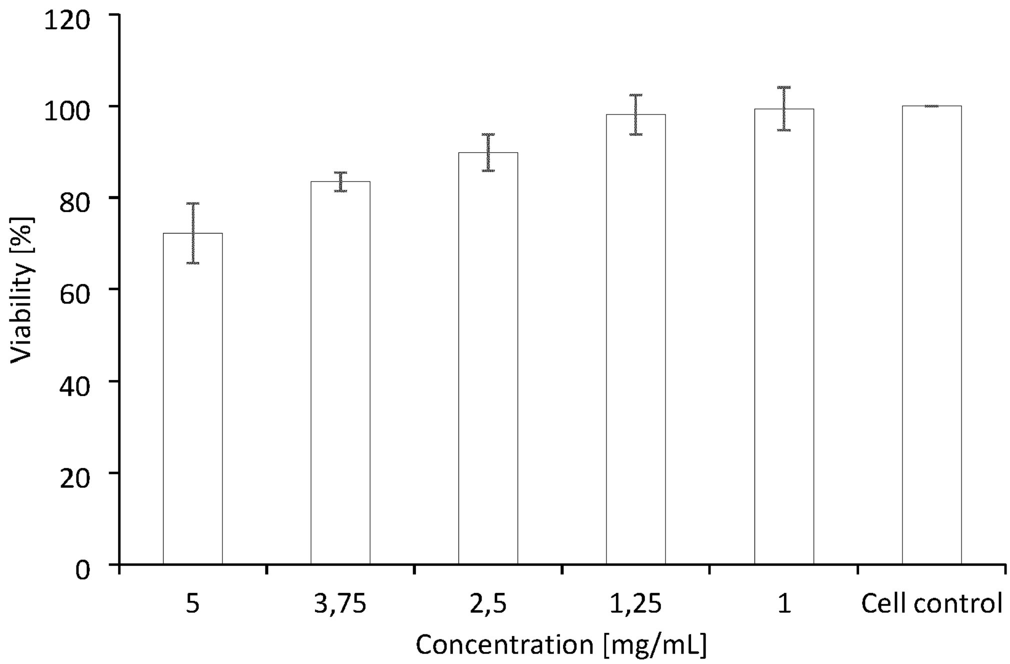
120
100
80
60
40
20
0
Viability [%]
5
3,75
2,5
1,25
1
Cell control
Concentration [mg/mL]

FIG 14

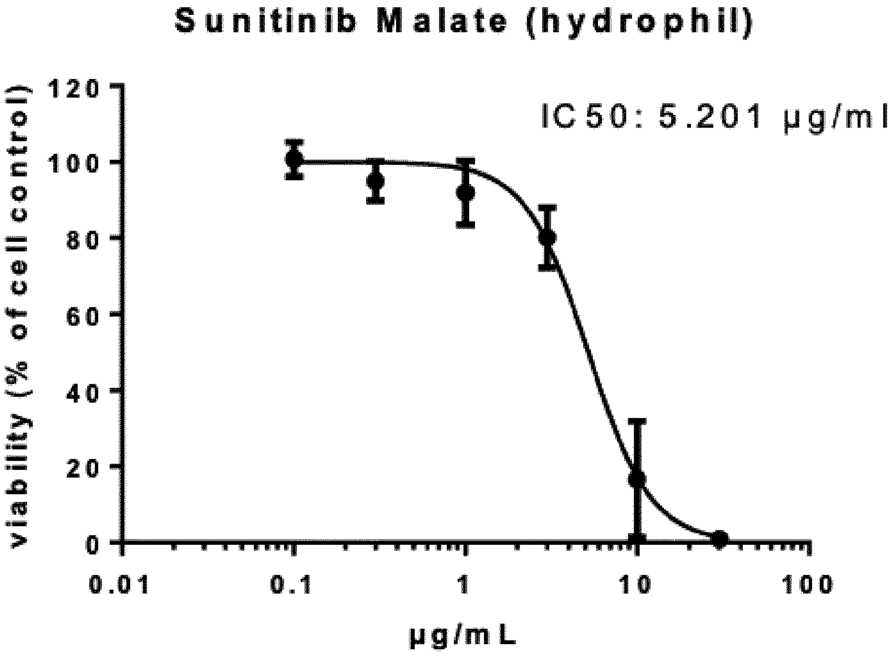


FIG 15A

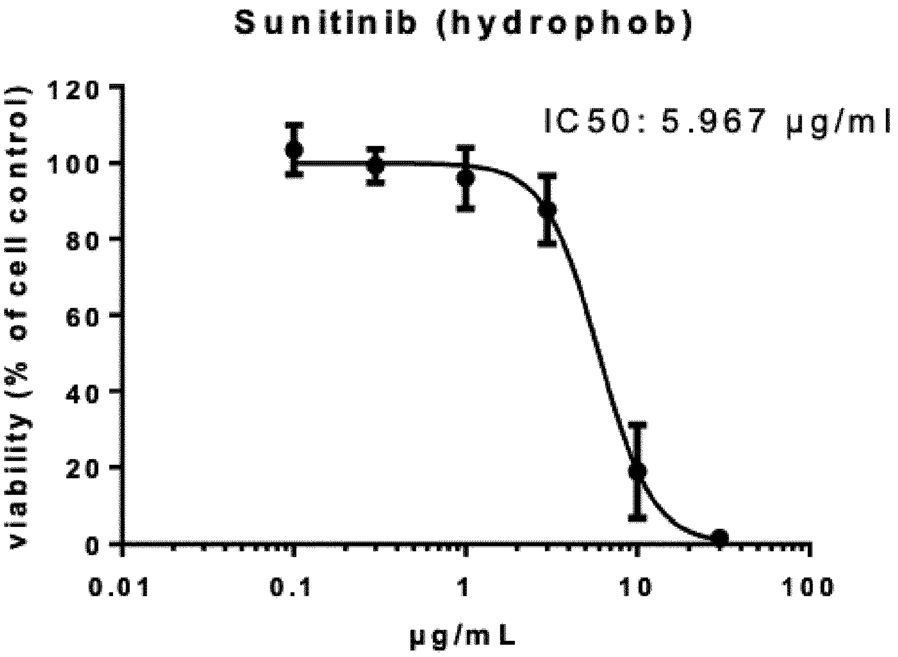


FIG 15B

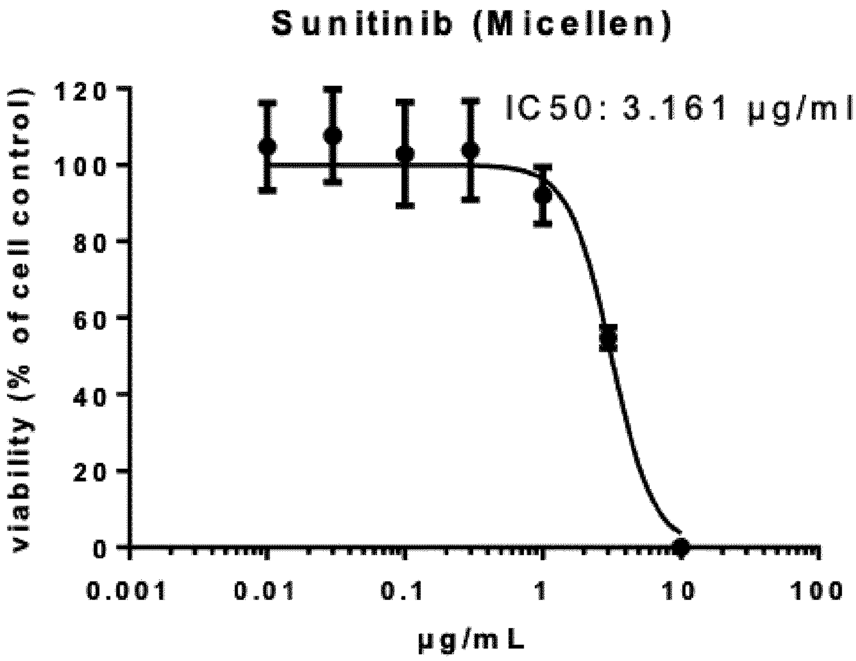


FIG 15C

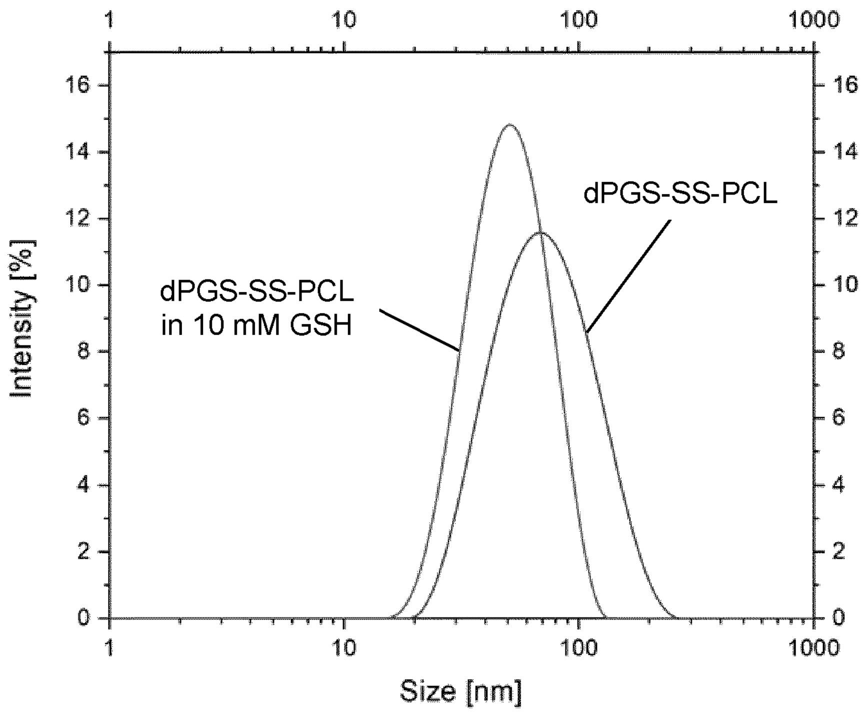


FIG 16

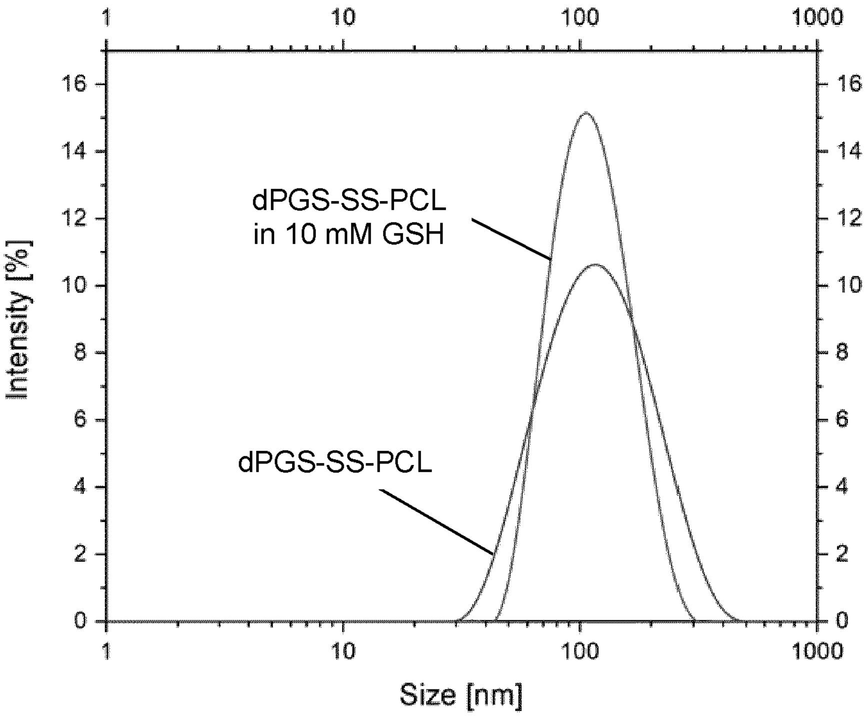
1
10
100
1000
16
14
12
10
8
6
4
2
0
Intensity [%]
dPGS-SS-PCL
in 10 mM GSH
dPGS-SS-PCL
Size [nm]

FIG 17

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **HAAG, R. ; STUMBÉ, J.-F. ; SUNDER, A ; FREY, H. ; HEBEL, A.** *Macromolecules,* 2000, vol. 33, 8158
- **WYSZOGRODZKA, M. ; HAAG, R.** *Biomacromolecules,* 2009, vol. 10, 1043
- **BAWA, K. K. ; JAZANI, A. M. ; SHETTY, C. ; OH, J. K.** *Macromolecular Rapid Communications,* 2018, vol. 39, 1800477
- **FERRARO, M. ; SILBERREIS, K. ; MOHAMMADIFAR, E. ; NEUMANN, F. ; DERNEDDE, J. ; HAAG, R.** *Biomacromolecules,* 2018, vol. 19, 4524
- **SKHIRI, Y. ; GRUNER, P. ; SEMIN, B. ; BROSSEAU, Q. ; PEKIN, D. ; MAZUTIS, L. ; GOUST, V. ; KLEINSCHMIDT, F. ; EL HARRAK, A. ; HUTCHISON, J. B.** *Soft Matter,* 2012, vol. 8, 10618
- **ZHONG Y ; DIMDE M ; STÖBENER D ; MENG F ; DENG C ; ZHONG Z ; HAAG R.** *ACS Appl Mater Interfaces,* 2016, vol. 8, 27530